(19) Europäisches Patentamt
European Patent Office
Office européen des brevets

(11) **EP 2 470 913 B1**

(12) **EUROPÄISCHE PATENTSCHRIFT**

(45) Veröffentlichungstag und Bekanntmachung des Hinweises auf die Patenterteilung:
**22.01.2020 Patentblatt 2020/04**

(21) Anmeldenummer: **10749831.3**

(22) Anmeldetag: **26.08.2010**

(51) Int Cl.:
*G01N 35/00* (2006.01)     *A61B 5/00* (2006.01)
*A61B 5/145* (2006.01)     *A61B 5/1495* (2006.01)
*G01N 27/327* (2006.01)     *G01N 33/66* (2006.01)
*G06F 17/18* (2006.01)

(86) Internationale Anmeldenummer:
**PCT/EP2010/062474**

(87) Internationale Veröffentlichungsnummer:
**WO 2011/023760 (03.03.2011 Gazette 2011/09)**

(54) **Kalibrierverfahren zur prospektiven Kalibrierung eines Messgeräts, Computerprogramm und Messgerät**

Calibration method for prospective calibration of a measuring device, computer program and measuring device

Procédé de calibrage destiné au calibrage prospectif d'un appareil de mesure, produit-programme d'ordinateur et appareil de mesure

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **27.08.2009 EP 09168797**

(43) Veröffentlichungstag der Anmeldung:
**04.07.2012 Patentblatt 2012/27**

(73) Patentinhaber:
• **Roche Diabetes Care GmbH**
**68305 Mannheim (DE)**
Benannte Vertragsstaaten:
**DE**
• **F. Hoffmann-La Roche AG**
**4070 Basel (CH)**
Benannte Vertragsstaaten:
**AL AT BE BG CH CY CZ DK EE ES FI FR GB GR HR HU IE IS IT LI LT LU LV MC MK MT NL NO PL PT RO SE SI SK SM TR**

(72) Erfinder:
• **SCHMITT, Nikolaus**
**69124 Heidelberg (DE)**
• **FRISCH, Gerhard**
**68535 Edingen-Neckarhausen (DE)**

(74) Vertreter: **Altmann Stößel Dick Patentanwälte PartG mbB**
**Dudenstrasse 46**
**68167 Mannheim (DE)**

(56) Entgegenhaltungen:
**EP-A1- 1 154 718      US-A1- 2002 106 709**
**US-A1- 2006 264 719      US-A1- 2006 281 985**
**US-A1- 2008 021 666      US-B1- 6 233 471**

• **PASSING H ET AL: "A new biometrical procedure for testing the equality of measurements from two different analytical methods: application of linear regression procedures for method comparison studies in clinical chemistry, part I", JOURNAL OF CLINICAL CHEMISTRY & CLINICAL BIOCHEMISTRY, BERLIN, DE, Bd. 21, Nr. 11, 1. Januar 1983 (1983-01-01), Seiten 709-720, XP008118228,**
• **Peter J Rousseeuw ET AL: "NEW STATISTICAL AND COMPUTATIONAL RESULTS ON THE REPEATED MEDIAN LINE", New directions in statistical data analysis and Robustness, 1 January 1993 (1993-01-01), pages 177-194, XP055434277, Basel, Boston, London ISBN: 978-3-7643-2923-5 Retrieved from the Internet: URL:https://wis.kuleuven.be/stat/robust/pa pers/publications-1993/rousseeuwnetanyahum ount-computrmline-ndsdar-1993.pdf [retrieved on 2017-12-12]**

- **ANDREW F SIEGEL: "Robust regression using repeated medians", BIOMETRIKA, BIOMETRIKA TRUST, LONDON, GB , vol. 69, no. 1 1 January 1982 (1982-01-01), pages 242-244, XP009502299, ISSN: 0006-3444, DOI: 10.1093/BIOMET/69.1.242 Retrieved from the Internet: URL:https://doi.org/10.1093/biomet/69.1.24 2 [retrieved on 1982-04-01]**

**Beschreibung**

Gebiet der Erfindung

Die Erfindung betrifft ein Verfahren zum Betrieb eines Messgeräts zur Erfassung mindestens eines Analyten in einer Körperflüssigkeit, ein Computerprogramm mit Programmcode zur Durchführung des Verfahrens sowie ein Messgerät zur Erfassung mindestens eines Analyten in einer Körperflüssigkeit, welches eingerichtet ist, um das erfindungsgemäße Verfahren durchzuführen. Derartige Verfahren und Vorrichtungen werden insbesondere in der Medizintechnik eingesetzt, um, beispielsweise im privaten Bereich oder im Bereich von Pflegeeinrichtungen oder Krankenhäusern, kontinuierlich oder diskontinuierlich einen oder mehrere Analyte in Körperflüssigkeiten wie beispielsweise Blut, interstitieller Flüssigkeit oder anderen Arten von Körperflüssigkeit, zu überwachen. Insbesondere kann das Verfahren zum Betrieb eines Messgeräts mit mindestens einem kontinuierlich messenden Blutglukose-Sensor eingesetzt werden. Derartige Sensoren, mittels deren ein so genanntes Continuous Monitoring durchgeführt werden kann, werden in der Regel für mehrere Tage in ein Fettgewebe oder interstitielles Gewebe eines Benutzers implantiert, um dann, beispielsweise in regelmäßigen oder unregelmäßigen Zeitabständen, Messsignale zu generieren, aus welchen auf die Konzentration des mindestens einen Analyten rückgeschlossen werden kann. Bei dem mindestens einen Analyten kann es sich insbesondere um Glukose, beispielsweise Blutglukose, handeln. Allgemein sind jedoch auch andere Anwendungen als die genannten und im Folgenden beschriebenen Anwendungen möglich.

Da es sich um implantierte Sensoren handelt, müssen die Systeme mit bereits etablierten Messverfahren kalibriert werden. Die Behandlung des Fehlers des Referenzsystems sowie die Erhebung der Kalibrationsdaten 'im Feld', d.h. ohne Norm, sind daher eine besondere Herausforderung an diese Systeme.

Stand der Technik

Messgeräte zur Erfassung mindestens eines Analyten in einer Körperflüssigkeit basieren in der Regel auf einem oder mehreren physikalischen und/oder chemischen Messprinzipien, mittels derer entsprechend ein oder mehrere Messsignale generiert werden. Beispielsweise kann es sich bei diesen Messprinzipien um elektrochemische Messprinzipien handeln, mittels derer eine oder mehrere Analytkonzentrationen nachweisbar sind. Derartige elektrochemische Messprinzipien sind aus dem Stand der Technik bekannt.

Eine Problematik derartiger Vorrichtungen besteht jedoch darin, dass die Messsignale zunächst in der Regel ohne physiologische Bedeutung sind. Beispielsweise kann es sich bei den Messsignalen um einfache Ströme, gemessen beispielsweise in Milliampere oder Nanoampere, handeln. Um eine physiologisch verwertbare Information aus diesen Messsignalen zu gewinnen, müssen diese Messsignale mit einer geeigneten Umrechnungsvorschrift in entsprechende Analytkonzentrationen umgerechnet werden. Diese Umrechnungsvorschrift, welche beispielsweise in einer Datenverarbeitungsvorrichtung hinterlegt sein kann, wird in der Regel auch als Kalibration bezeichnet.

Zur Anwendung der Umrechnungsvorschrift sind in der Regel charakteristische Kenngrößen (Parameter) notwendig. Diese können, da der Sensor vom Patienten implantiert und daher die Messumgebung nicht exakt definiert ist, nicht alle im Vorfeld definiert werden. Daher ist es notwendig, während der Messung Vergleichsmessungen durchzuführen, um die Messsignale in die Analytkonzentration mit hoher Genauigkeit umrechnen zu können, wobei keine absolute Norm bzw. Referenz zur Verfügung steht.

Bei einer Vergleichsmessung im Vorfeld wird die Konzentration des mindestens einen Analyten mittels eines oder mehrerer Referenzverfahren bestimmt. Die Erfindung wird im Folgenden im Wesentlichen unter Bezugnahme auf Blutglukose als Analyt beschrieben. Alternativ oder zusätzlich können jedoch auch andere Arten von Analyten bestimmt werden. Bei der Bestimmung von Blutglukose kann beispielsweise die Blutglukose direkt mittels eines chemischen Nachweisverfahrens bestimmt werden und/oder mittels einer anderen Art von Referenzmessung, deren Kalibration bereits bekannt ist. Für die Kalibration werden dann die Messsignale des Messgeräts mit den Referenzwerten der Referenzmessungen in Beziehung zueinander gesetzt. Beispielsweise können dies gemessene Stromkurven kontinuierlich messender Glukosesysteme sein (Continuous Monitoring Systems), die in Beziehung zu auf andere Weise gemessenen Blutglukosemessungen, beispielsweise Einzelmessungen mittels Teststreifen, gesetzt werden. Aus dieser bekannten Beziehung, welche in eine entsprechende Kalibration einfließt, kann dann bei zukünftigen Messungen aus Messsignalen des Messgeräts auf eine Konzentration des Analyten in der Körperflüssigkeit geschlossen werden. Zu beachten ist hierbei, dass auch die Referenzmessungen einen nicht zu vernachlässigenden Messfehler besitzen.

Aus H. Passing und W. Bablok: A New Biometrical Procedure for Testing the Equality of Measurements from Two Different Analytical Methods: Application of linear regression procedures for method comparison studies in Clinical Chemistry, part I, Journal of Clinical Chemistry Clinical Biochemistry, Vol.21, 1983, Seiten 709-720, ist allgemein ein biometrisches Verfahren zur Überprüfung der Gleichheit von Messwerten zweier analytischer Methoden bekannt. Hier wird retrospektiv die Verwendung von linearen Regressionsverfahren bei Methodenvergleichsstudien in der klinischen Chemie beschrieben.

Aus dem Stand der Technik ist eine Vielzahl von Kalibrationsverfahren für Messgeräte, insbesondere für Glukosemessgeräte, bekannt. Im Folgenden wird dabei, ohne Beschränkung möglicher weiterer Anwendungen, insbesondere Bezug genommen auf Messgeräte, welche mindestens einen kontinuierlich messenden Sensor, insbesondere mindestens einen kontinuierlich messenden Blutglukose-Sensor, umfassen.

Viele der bekannten Verfahren zur Kalibrierung stellen eine Korrelation zwischen dem Messsignal und dem Glukoseverlauf im Blut durch verschiedene Standard-Regressionsverfahren her. Beispielsweise kann es sich dabei um eine lineare Regression, Anpassungen nach der Methode der kleinsten Fehlerquadrate oder Ähnliches handeln. Ein Beispiel derartiger linearer Regressionsverfahren ist in EP 1 154 718 B1 dargestellt. Dort werden abgetastete Daten einer Glukoseüberwachungsvorrichtung unter Verwendung mindestens einer Blutglukosereferenzablesung kalibriert. Insbesondere wird vorgeschlagen, eine Berechnung von Kalibrierfaktoren unter Verwendung einer linearen Regression vorzunehmen.

Eine weitere Methode zur Berechnung eines Zusammenhangs zwischen den Messsignalen und den Referenzwerten ist die Verwendung von Expertensystemen. Diese werden beispielsweise in US 6,326,160 B1 oder in US 6,233,471 erwähnt. Diese Methoden verwenden gewichtete Summen, um eine Korrelation zwischen kontinuierlich gemessenem Stromverlauf und Blutglukoseverlauf herzustellen.

US 2006/264719 A1 offenbart ein Verfahren zum Betrieb eines Messgeräts zur Erfassung mindestens eines Analyten in einer Körperflüssigkeit mittels mindestens eines kontinuierlich messenden Blutglukose-Sensors, wobei mindestens ein Kalibrierverfahren des Messgeräts durchgeführt wird.

Aus US 2008/0081977A1 ist ebenfalls ein Kalibrationsmodell bekannt, welches insbesondere auch Versatzzeiten zwischen der Erfassung der Referenzwerte und der Messsignale berücksichtigt.

In US 2008/0021666 A1 wird ebenfalls die Auftragung von Kalibrationsdaten gegen gemessene Daten vorgenommen. Auch in dieser Schrift wird ein Regressionsverfahren (hier least square regression, Methode der kleinsten Fehlerquadrate) vorgenommen, und es wird auf diese Weise eine Steigung einer angepassten Geraden durch die Kalibrationspunkte berechnet.

Aus US 2006/0281985A1 ist ein Verfahren zur Kalibrierung eines Biosensors zur Erfassung eines Analyten bekannt. Dabei wird über einen Zeitraum hinweg eine Vielzahl von Messsignalen des Biosensors erfasst. Ein Median-Filter wird auf diese Vielzahl von Messsignalen angewandt, und der so erhaltene Median-Wert wird eingesetzt, um, aus einem Vergleich mit einer gemessenen Blut-Analytkonzentration, eine Sensor-Sensitivität zu ermitteln. Dabei können für unterschiedliche Phasen unterschiedliche Wichtungen der Sensitivitäten vorgenommen werden.

Weiterhin stellt sich in vielen Fällen bei realen Messungen, insbesondere bei kontinuierlichen Messungen, welche über einen längeren Zeitraum hin erfolgen, die Problematik einer Handhabung der Vielzahl von Daten. Zu diesem Zweck wurden Datenkompressionsverfahren entwickelt, wie beispielsweise in US 7,389,133 B1 oder US 2007/0016127 A1 beschrieben.

Der dargestellte Stand der Technik weist jedoch in der praktischen Handhabung eine Vielzahl von Nachteilen und technischen Herausforderungen auf. So nehmen beispielsweise die meisten der beschriebenen Kalibrationsverfahren in keiner Weise oder lediglich unzureichend Rücksicht auf das Auftreten von Ausreißern. So können beispielsweise bei der Kalibration außergewöhnliche Kalibrationspunkte auftreten, also Kalibrationspunkte, welche weit außerhalb des nach den übrigen Kalibrationspunkten erwarteten Verlaufs oder Wertebereichs liegen und umgangssprachlich als Ausreißer bezeichnet werden. Diese können zwar im Rahmen einer Plausibilitätsbetrachtung verworfen werden, was jedoch wieder zu einer Verfälschung der Kalibration führen kann.

Verfahren zur Umrechnung der Messsignale in interpretierbare Analytkonzentrationen, beispielsweise Blutglukosewerte, sollten allgemein den Verlauf der Analytkonzentration möglichst genau wiedergeben. Zudem sollten diese robust sein gegenüber Ausreißern, die durch zufällige und systematische Störeinflüsse wie Temperatur, Bewegung des Benutzers (beispielsweise eines Patienten) oder ähnliche Störeinflüsse, entstehen. Auch wird der Einfluss eines Messfehlers in der Referenzmessung nicht berücksichtigt. Die bekannten Verfahren berücksichtigen diese Anforderungen nur unzureichend. Ein wesentlicher Punkt ist dabei in vielen Fällen, dass das Messsignal stark abhängig sein kann von der Lage und/oder Positionierung des eigentlichen Sensors und beispielsweise bei implantierten Sensoren von Insertionseinflüssen. Eine Vorkalibration, wie sie bei Teststreifensystemen üblich ist, scheidet daher in der Regel aus.

Zur Kalibration der Messsignale kontinuierlich messender Sensoren werden daher in vielen Fällen sporadisch, beispielsweise im Abstand von zwölf Stunden, Spot-Messungen herangezogen, bei welchen die Kalibration beispielsweise gegen einen oder mehrere mittels eines Teststreifengeräts gemessene Referenzwerte erfolgt. Dieses Vorgehen birgt jedoch einige Herausforderungen an die Kalibration in sich. So sind beispielsweise ein Blutglukosespiegel, wie er von Spot-Monitoring-Systemen gemessen werden kann, und Interstitium-Glukosespiegel, wie er beispielsweise mit einem kontinuierlich messenden, implantierten Sensor gemessen werden kann, zwar stark miteinander korreliert, sind jedoch zunächst verschiedene Messgegenstände. Insbesondere können zwischen dem Blutglukoseverlauf und dem Interstitium-Glukoseverlauf Totzeiten auftreten, welche nicht notwendigerweise konstant sind. Zudem können die zur Messung herangezogenen Blutglukosewerte in der Praxis kaum kontrolliert werden. Während in normalen Kalibrationsverfahren Vorgaben bezüglich des abgedeckten Konzentrationsbereichs existieren, ist man hier in vielen Fällen darauf angewiesen, die vom Patienten vorgegebenen Konzentrationsbereiche zu verarbeiten. Dementsprechend kann es sein, dass der Konzentrationsbereich, innerhalb dessen eine Kalibration durchgeführt werden kann, vergleichsweise klein ist. Dementsprechend ungenau können Messungen später sein, wenn Messsignale außerhalb des kalibrierten Bereichs auftreten. Weiterhin ist die Anzahl der vorhandenen Kalibrationspunkte in der Praxis stark beschränkt, und diese Kalibrationspunkte bergen eine starke Unsicherheit in sich, da diese in der Regel nicht mit Referenzmessungen unter Laborbedingungen, sondern mittels Referenzmessungen mit einfachen Alltags-Blutglukosegeräten und unter undefinierten Messbedingungen durchgeführt werden. Ebenso liegt die vollständige Anzahl der Kalibrationspunkte in der Regel erst am Ende der Messzeit vor. Der Benutzer möchte jedoch in der Regel so früh wie möglich über den Verlauf seines Glukosespiegels informiert werden.

Aufgabe der Erfindung

Es ist daher eine Aufgabe der Erfindung, ein Verfahren zum Betrieb eines Messgeräts sowie ein entsprechendes Messgerät anzugeben, welche die Nachteile bekannter Verfahren und Messgeräte vermeiden. Insbesondere soll ein gegenüber Ausreißern und Referenzmessfehlern robustes Kalibrierverfahren bereitgestellt werden, welches auch unter in der Praxis vorherrschenden Bedingungen eine zuverlässige Auswertung von Messwerten ermöglicht.

Offenbarung der Erfindung

Diese Aufgabe wird gelöst durch ein Verfahren, ein Messgerät und ein Computerprogramm mit den Merkmalen der unabhängigen Ansprüche. Vorteilhafte Weiterbildungen der Erfindung, welche einzeln oder in Kombination realisiert werden können, sind in den abhängigen Patentansprüchen dargestellt.

In einem ersten Aspekt der Erfindung wird ein Verfahren zum Betrieb eines Messgeräts zur Erfassung mindestens eines Analyten in einer Körperflüssigkeit vorgeschlagen. Wie oben dargestellt, kann dieses Messgerät insbesondere die Erfassung des Analyten mittels mindestens eines kontinuierlich messenden Blutglukose-Sensors vornehmen. Auch andere Arten von Sensoren, beispielsweise Sensoren, welche alternativ oder zusätzlich andere Arten von Analyten erfassen, und/oder nicht-kontinuierlich messende Sensoren, sind jedoch grundsätzlich einsetzbar.

Bei dem Verfahren wird mindestens ein Kalibrationsverfahren zur prospektiven Kalibrierung des Messgeräts durchgeführt. Unter einer prospektiven Kalibrierung ist dabei die Ermittlung einer Umrechnungsvorschrift zu verstehen, welche für zukünftige Messungen die Umrechnung von Messsignalen des Messgeräts, beispielsweise mindestens eines Sensors des Messgeräts, in eine Konzentration des Analyten in der Körperflüssigkeit ermöglicht. Zusätzlich zu mindestens einer prospektiven Kalibrierung kann jedoch, wie unten noch näher ausgeführt wird, auch optional mindestens eine retrospektive Kalibrierung durchgeführt werden, bei welcher nachträglich Messsignale neu ausgewertet und aufgrund nachträglich gewonnener Erkenntnisse erneut in Analytkonzentrationen umgerechnet werden.

In dem Kalibrierverfahren werden mindestens drei Kalibrationspunkte erfasst. Vorzugsweise können dabei mehr als drei Kalibrationspunkte erfasst werden. Beispielsweise können, wie unter näher ausgeführt wird, iterativ neue Kalibrationspunkte hinzugenommen und bei dem Kalibrierverfahren berücksichtigt werden. Ältere Kalibrationspunkte können optional verworfen oder geringer gewichtet werden. Jeder Kalibrationspunkt enthält dabei mindestens ein Messsignal des Messgeräts und mindestens einen Referenzwert einer zugehörigen Referenzmessung. Dies bedeutet, dass zur Ermittlung eines Kalibrationspunkts einmal mindestens ein Messsignal des Messgeräts erfasst wird. Gleichzeitig oder mit einem möglichst geringen Zeitversatz (beispielsweise einem Zeitversatz von nicht mehr als 5 Minuten, vorzugsweise von nicht mehr als 1 Minute) wird mittels einer Referenzmessung mindestens ein Referenzwert erfasst, welcher eine Information über eine tatsächlich vorhandene Analytkonzentration in der Körperflüssigkeit liefert. Auch der Referenzwert kann naturgemäß fehlerbehaftet sein und sollte dementsprechend lediglich eine möglichst genaue Schätzung bzw. Bestimmung der tatsächlich vorhandenen Analytkonzentration in der Körperflüssigkeit darstellen, welche über die Referenzmessung erhalten wird. Die Referenzmessung sollte dementsprechend mittels mindestens eines Verfahrens durchgeführt werden, welches bekanntermaßen Messergebnisse liefert, die so nahe wie möglich an der tatsächlich vorhandenen Analytkonzentration liegen. Beispielsweise kann diese Referenzmessung eine Blutglukosemessung mittels eines anderen Verfahrens umfassen, beispielsweise eine Spot-Messung, bei welcher einmalig eine Probe der Körperflüssigkeit untersucht wird. Beispielsweise kann die Referenzmessung eine Messung mit einem Handgerät umfassen. Alternativ oder zusätzlich kann die Referenzmessung jedoch grundsätzlich auch eine Labormessung umfassen. Im Folgenden wird insbesondere auf die Referenzmessung mittels eines oder mehrerer Handgeräte Bezug genommen, insbesondere mittels eines oder mehrerer Teststreifen, beispielsweise unter Verwendung eines optischen und/oder eines elektrochemischen Nachweisverfahrens.

Die Messsignale und die Referenzwerte werden zu Kalibrationspunkten zusammengefasst. Darüber hinaus können die Kalibrationspunkte weitere Informationen umfassen. Die Kalibrationspunkte können beispielsweise in einem Datenspeicher abgelegt werden, beispielsweise in einem Datenspeicher eines Computers einer Steuerung des Messgeräts.

Bei den Messsignalen der Kalibrationspunkte kann es sich grundsätzlich um Roh-Messsignale handeln, welche mit dem Messgerät erfasst wurden. Alternativ kann es sich jedoch bei diesen Messsignalen auch bereits um zumindest teilweise aufbereitete Messsignale handeln, beispielsweise Messsignale, welche über einen Zeitraum hinweg erfasst wurden, und welche anschließend einer Datenaufbereitung unterzogen wurden, beispielsweise einer Filterung, einer Mittelwertbildung, einer Glättung oder einer Linearisierungstransformation. Eine Linearisierungstransformation bildet ein nichtlineares Verhältnis zwischen Messignalen und Konzentration des Analyten auf eine lineares Verhältnis ab. In der linearisierten Darstellung wird die erfindungsgemäße Kalibrierung durchgeführt, wobei zur Darstellung der Konzentration eine dazu komplementäre Transformation ausgeführt werden kann. Die Linearisierungstransformation gibt eine angenäherte Abhängigkeit zwischen Messignalen und Konzentration wieder, die Eigenschaften des Messsystems widerspiegelt. Beispielsweise kann durch Sättigung bei hohen Konzentrationen die Empfindlichkeit des Messsystems mit zunehmender Konzentration verringern. Insbesondere für diesen Bereich kann eine logarithmische Abhängigkeit zwischen Messsignal und Konzentration angenommen werden. Eine Linearisierungstransformation transformiert die Messsignale insbesondere in diesem Bereich gemäß dieser Abhängigkeit, um (angenähert) einen linearen Zusammenhang zwischen Messsignal und Konzentration zu erreichen. Die Kalibration wird mit den linearisierten Werten des Messsignals vorgenommen, um die zugrunde liegende Betrachtung der möglichen Steigungen zwischen Messpunkten ohne einen Fehler ausführen zu können, der von einem nichtlinearen Zusammenhang stammt. Die Linearisierungstransformation entspricht der Umkehrfunktion des Zusammenhangs zwischen Messsignal und Konzentration. Eine komplementäre Transformation zur Umwandlung des Messsignals in Konzentrationswerte entspricht wiederum der Umkehrfunktion der Linearisierungstransformation und somit dem Zusammenhang zwischen Messsignal und Konzentrationswerte, der sich aus den Eigenschaften des Systems ergibt. Die Linearisierungstransformation kann nur ein Intervall des Messsignals betreffen (beispielsweise ein nach oben offenes Intervall, das einen Sättigungsbereich des Messsystems entspricht) oder kann den gesamten Bereich des Messsignals betreffen. Die Linearisierungstransformation kann ferner eine empirisch gewonnene Funktion sein, die die Eigenschaften des Messsystems empirisch wiedergibt oder eine Funktion, die mindestens einen physikalischen Prozess abbildet, beispielsweise eine Sensorsättigung. Die Linearisierungstransformation kann ferner durch ein Polynom oder ein Spline dargestellt werden oder durch eine Reihenentwicklung, wobei die zugehörigen Koeffizienten die Transformation bestimmen.

Eine derartige Transformation kann auch verwendet werden, um eine zeitliche Veränderung (Degeneration) des Sensors auszugleichen. Dabei kann diese Transformation unabhängig von dem oben beschriebenen Linearisierungsschritt durchgeführt werden. Alternativ können die in diesem Abschnitt und die in den vorangehenden Abschnitt dargestellte Transformationen kombiniert durchgeführt werden, d.h. zeitlicher Drift und Nichtlinearität können mit einer zweidimensionalen Transformation in einem (gemeinsamen) Schritt durchgeführt werden.

Im Rahmen einer Datenaufbereitung ist auch die Bildung eines Medians möglich. Gleiches gilt auch für die Referenzwerte. Sind mehrere Referenzwerte vorhanden, so können auch diese einer Datenverarbeitung unterzogen werden. Ist allgemein eine derartige Datenaufbereitung vorgesehen, so werden die aufbereiteten Messsignale bzw. die aufbereiteten Referenzwerte zusammengefasst zu den jeweiligen Kalibrationspunkten. Jeder Kalibrationspunkt umfasst also zumindest ein Messsignal, gegebenenfalls ein aufbereitetes Messsignal, und zumindest einen Referenzwert, gegebenenfalls einen aufbereiteten Referenzwert.

Sind die mindestens drei Kalibrationspunkte erfasst, so wird eine Mehrzahl möglicher Steigungen zwischen den Kalibrationspunkten ermittelt. Sind beispielsweise drei Kalibrationspunkte vorhanden, so sind dies 2*3/2=3 Steigungen. Bei n Kalibrationspunkten sind dies n*(n-1)/2 mögliche Steigungen. Dabei können alle Kalibrationspunkte berücksichtigt werden, sodass sämtliche mögliche Steigungen zwischen den Kalibrationspunkten ermittelt werden. Alternativ können jedoch auch eine oder mehrere der möglichen Steigungen bei dem Kalibrierverfahren ignoriert werden, sodass weniger als die genannte Maximalanzahl möglicher Steigungen ermittelt werden. In jedem Fall sollten jedoch mindestens zwei Steigungen zwischen Kalibrationspunkten ermittelt werden, vorzugsweise mindestens drei Steigungen und besonders bevorzugt mehr als drei Steigungen. Es wird jedoch darauf hingewiesen, dass das Kalibrierverfahren optional auch zunächst damit begonnen werden kann, dass lediglich ein einzelner Kalibrationspunkt vorliegt. Als weiterer Kalibrationspunkt kann dann beispielsweise zunächst der Nullpunkt angenommen werden, welcher optional später wieder verworfen werden kann. Durch diese beiden Punkte, also einen gemessenen Kalibrationspunkt und einen angenommenen Kalibrationspunkt, welcher beispielsweise der Nullpunkt sein kann, kann dann eine Gerade definiert werden, deren Steigung und Achsenabschnitt (bei Wahl des Nullpunkts als gewählter Kalibrationspunkt ist dieser naturgemäß gleich 0) bestimmt werden können. Alternativ oder zusätzlich kann die Kalibration optional auch mit zunächst zwei Kalibrationspunkten begonnen werden, welche eine Gerade definieren. Die Steigung a und der Achsenabschnitt b dieser Geraden können ebenfalls bestimmt werden.

Aus den auf diese Weise ermittelten möglichen Steigungen zwischen den Kalibrationspunkten wird dann mittels mindestens eines robusten Schätzverfahrens unter Verwendung einer Bildung mindestens eines Medians, mindestens ein Schätzer für die Steigung der Kalibrationsgeraden bestimmt, die im folgenden als wahrscheinliche Steigung bezeichnet wird. Unter einem robusten Schätzverfahren ist dabei ein statistisches Schätzverfahren zu verstehen, welches auch bei dem Auftreten von Ausreißern oder nur approximativer Gültigkeit von Verteilungsannahmen noch stabile statistische Schätzer liefert.

Derartige robuste Schätzverfahren sind dem Fachmann grundsätzlich bekannt, beispielsweise aus der eingangs genannten Veröffentlichung von H. Passing und W. Bablok, dort allerdings im Rahmen eines retrospektiven Methodenvergleichs. Die dort beschriebenen robusten Schätzverfahren können jedoch grundsätzlich auch im Rahmen der vorliegenden Erfindung angewandt werden. Dabei können auch verschiedene robuste Schätzverfahren kombiniert werden. Insbesondere lassen sich robuste Schätzverfahren einsetzen, welche auf einem oder mehreren Permutationsalgorithmen und/oder einem oder mehreren Sortieralgorithmen basieren, also Algorithmen, bei welchen Werte der Größe nach geordnet werden. Ein besonders bevorzugtes Ausführungsbeispiel eines robusten Schätzverfahrens und insbesondere eines Sortieralgorithmus ist ein Schätzverfahren, welches eine Bildung mindestens eines Medians verwendet. Dementsprechend ist es besonders bevorzugt, wenn aus der Mehrzahl möglicher Steigungen die mindestens eine wahrscheinliche Steigung mittels einer Median-Bildung bestimmt wird. Sind beispielsweise n Kalibrationspunkte bestimmt worden und dementsprechend n*(n-1)/2 Steigungen oder weniger, mindestens jedoch zwei, vorzugsweise drei, vier oder mehr Steigungen, so kann aus diesen möglichen Steigungen die wahrscheinliche Steigung als Median der ermittelten Steigungen bestimmt werden.

Der Median einer Messreihe ist dabei ein Zahlenwert, für den mindestens die Hälfte aller Beobachtungen der Messreihe kleiner oder gleich dem Median sind und mindestens die Hälfte aller Beobachtungen der Messreihe größer oder gleich dem Median sind. Aus dieser Definition folgt, dass einzelne Ausreißer keinen Einfluss auf den Median haben. Der Median ist daher ein robuster Schätzer für den Erwartungswert einer Zufallsgröße. Bei einer Menge von möglichen Steigungen $\{a_1, a_2, ... a_n\}$, welche der Größe nach sortiert sind, wird als Median typischerweise der Wert $a_{(n+1)/2}$ verwendet, wenn n ungerade ist, und das arithmetische Mittel $\frac{1}{2}\left(a_{(n/2)} + a_{(n/2)+1}\right)$ wenn n gerade ist. Grundsätzlich können, alternativ oder zusätzlich zur Verwendung der Bildung des Medians, auch andere robuste Schätzverfahren eingesetzt werden, insbesondere andere robuste Schätzverfahren, welche auf einem oder mehreren Permutationsalgorithmen und/oder einem oder mehreren Sortieralgorithmen basieren und/oder andere Arten robuster Schätzverfahren. Auch eine Kombination mehrerer unterschiedlicher robuster Schätzverfahren ist einsetzbar.

Weiterhin wird in dem vorgeschlagenen Verfahren mindestens eine Messung durchgeführt. Bei dieser Messung wird unter Verwendung der wahrscheinlichen Steigung aus mindestens einem Messsignal des Messgeräts und der wahrscheinlichen Steigung auf eine Konzentration des Analyten in der Körperflüssigkeit geschlossen. Beispielsweise kann zu diesem Zweck ein Offset oder ein Achsenabschnitt angenommen werden, welcher beispielsweise auch aus einem Erfahrungswert gewonnen werden kann oder welcher willkürlich angenommen werden kann, beispielsweise als Null. Alternativ oder zusätzlich kann auch ein wahrscheinlicher Achsenabschnitt verwendet werden, welcher gemäß dem optionalen, unten beschriebenen Verfahren bestimmt wird. Dieses mindestens eine für die Messung verwendete Messsignal des Messgeräts kann bereits für das Kalibrierverfahren verwendet worden sein. Alternativ oder zusätzlich können jedoch separate Messsignale während der Messung aufgenommen werden, aus denen dann auf die Konzentration des Analyten in der Körperflüssigkeit geschlossen wird. Wie oben ausgeführt, kann es sich bei diesen Messsignalen beispielsweise um einen oder mehrere Ströme handeln, welche beispielsweise mittels mindestens einen amperometrischen Sensors des Messgeräts gewonnen wurden, beispielsweise unter Verwendung eines oder mehrerer elektrochemischer Nachweisverfahren zum Nachweis von Glukose in Blut, interstitieller Flüssigkeit oder ähnlichen Körperflüssigkeiten.

Der Rückschluss aus dem Messsignal des Messgeräts auf eine Konzentration des Analyten in der Körperflüssigkeit kann beispielsweise durch eine Umkehrung des bei dem Kalibrierverfahren festgestellten Zusammenhangs zwischen den Referenzwerten und den Messsignalen erfolgen. Eine derartige Umkehrung ist beispielsweise durch Bildung einer Umkehrfunktion in der Regel auf einfache Weise möglich, zumindest bei eineindeutigen Funktionen. Die wahrscheinliche Steigung kann dabei beispielsweise als so genannte Sensitivität verwendet werden und kann zur Bildung der Umkehrfunktion invertiert werden, um aus dem mindestens einen Messsignal aus der mindestens einen Messung auf die Konzentration des Analyten in der Körperflüssigkeit zu schließen.

Das Verfahren kann dabei wiederholt durchgeführt werden und/oder derart, dass die beiden genannten Verfahrensschritte des Kalibrierverfahrens und der Referenzmessung zeitlich parallel, zeitlich überlappend oder verteilt durchgeführt werden.

In einer besonders vorteilhaften Ausgestaltung werden bei dem Kalibrierverfahren weiterhin eine Mehrzahl von Geraden bestimmt, deren Steigung der wahrscheinlichen Steigung entspricht und jeweils einen Kalibrationspunkt enthält. Vorzugsweise wird diese Mehrzahl der Geraden durch alle Kalibrationspunkte bestimmt, wobei jedoch auch eine geringere Anzahl als die maximal vorhandene Anzahl der Kalibrationspunkte verwendet werden kann. Insgesamt wird auf diese Weise eine Mehrzahl paralleler Geraden, welche jeweils die wahrscheinliche Steigung aufweisen, gebildet, welche durch die Mehrzahl der Kalibrationspunkte verläuft. Diese Geraden schneiden die Achsen, beispielsweise die y-Achse, in der Regel in unterschiedlichen Punkten. Es werden jeweils die Achsenabschnitte der Geraden bestimmt. Unter einem Achsenabschnitt ist dabei der Schnittpunkt der Geraden mit der y-Achse zu verstehen. Ebenfalls unter diesem Begriff subsumiert werden kann jedoch auch eine andere Größe, aus welcher, gegebenenfalls unter Zuhilfenahme der wahrscheinlichen Steigung, auf den Achsenabschnitt geschlossen werden kann, beispielsweise der Schnittpunkt mit der x-Achse. Welche Achse in dieser Darstellung x-Achse und welche Achse y-Achse ist, hängt von der Art der Auftragung ab und kann grundsätzlich variieren. So kann als x-Achse beispielsweise der Blutglukose-Wert bzw. der Wert der Konzentration des Analyten in der Körperflüssigkeit verwendet werden, und als y-Achse das Messsignal des Messgeräts, oder umgekehrt.

Mittels des vorgeschlagenen Verfahrens wird also vorzugsweise eine Mehrzahl von Achsenabschnitten der Geraden bestimmt. Anschließend kann mittels mindestens eines robusten Schätzverfahrens aus diesen Achsenabschnitten mindestens ein wahrscheinlicher Achsenabschnitt bestimmt werden. Dabei kann es sich grundsätzlich um dasselbe robuste Schätzverfahren wie bei der Bestimmung der wahrscheinlichen Steigung handeln oder grundsätzlich auch um eine andere Art eines robusten Schätzverfahrens. Besonders bevorzugt ist insbesondere jedoch wieder die Verwendung einer Bildung mindestens eines Medians der Achsenabschnitte zur Bestimmung eines statistischen Schätzers des Achsenabschnitts, der im Folgenden als wahrscheinlicher Achsenabschnitt bezeichnet wird. Alternativ oder zusätzlich könnten jedoch allgemein beispielsweise auch andere Algorithmen eingesetzt werden, welche auf einem oder mehreren Permutationsalgorithmen und/oder einem oder mehreren Sortieralgorithmen basieren, aus denen die Bildung eines Medians lediglich ein bevorzugtes Beispiel darstellt.

Ist auf diese Weise der wahrscheinliche Achsenabschnitt bestimmt, so wird bei der Messung der wahrscheinliche Achsenabschnitt zusätzlich zu der wahrscheinlichen Steigung verwendet, um aus dem Messsignal des Messgeräts auf die Konzentration des Analyten in der Körperflüssigkeit zu schließen. Mit dem wahrscheinlichen Achsenabschnitt und der wahrscheinlichen Steigung ist ein eindeutig vorgegebener linearer Zusammenhang zwischen den Analytkonzentrationen in der Körperflüssigkeit und den Messsignalen des Messgeräts vorgegeben, welcher auch auf einfache Weise invertiert werden kann. Es sei jedoch darauf hingewiesen, dass der wahrscheinliche Achsenabschnitt auch auf andere Weise als unter Verwendung eines robusten Schätzverfahrens ermittelt werden kann, beispielsweise durch ein nicht-robustes Schätzverfahren, beispielsweise ein parametrisches Schätzverfahren. Beispielsweise kann eine einfache Mittelwertbildung, beispielsweise unter Bildung eines arithmetischen Mittelwerts, aus den Achsenabschnitten erfolgen, und/oder es kann eine andere Art von Geraden durch die Kalibrationspunkte verwendet werden, welche die wahrscheinliche Steigung aufweist.

Das Kalibrierverfahren kann, wie oben dargestellt, insbesondere wiederholt durchgeführt werden, insbesondere zu verschiedenen Zeiten. Dabei kann nach einer Wiederholung des Kalibrierverfahrens insbesondere eine neue wahrscheinliche Steigung und vorzugsweise ein neuer wahrscheinlicher Achsenabschnitt bestimmt werden. Diese neue wahrscheinliche Steigung und vorzugsweise der neue wahrscheinliche Achsenabschnitt können dann anstelle der zuvor verwendeten wahrscheinlichen Steigung beziehungsweise des zuvor verwendeten wahrscheinlichen Achsenabschnitts in mindestens einer nachfolgenden Messung verwendet werden, um aus mindestens einem Messsignal wiederum auf die Konzentration des Analyten in der Körperflüssigkeit zu schließen.

Das vorgeschlagene Verfahren kann weiterhin mit bekannten Verfahren kombiniert werden. So kann beispielsweise bei dem Kalibrierverfahren eine Plausibilitätsbetrachtung durchgeführt werden. Bei der Plausibilitätsbetrachtung handelt es sich dabei um eine Betrachtung, bei welcher Werte, die außerhalb eines vorgegebenen Bereichs liegen, nicht berücksichtigt werden. Beispielsweise kann es sich bei dem vorgegebenen Bereich um Erfahrungswerte handeln. So können bei der Plausibilitätsbetrachtung unrealistische Kalibrationspunkte und/oder unrealistische Steigungen und/oder unrealistische Achsenabschnitte verworfen werden. Auch kann die Berechnung iterativ mit mehreren Untermengen der Kalibrationspunkte ausgeführt werden, zum Beispiel mit einer ‚leaving-one-out'-Methode, um die Abhängigkeit der wahrscheinlichen Steigung bzw. des wahrscheinlichen Offsets von einem Kalibrationspunkt zu untersuchen.

Bei dem vorgeschlagenen Verfahren kann weiterhin mindestens eine aktuelle Kalibrationsqualität bestimmt werden. Unter einer Kalibrationsqualität ist grundsätzlich mindestens ein Indikator zu verstehen, welcher angibt, mit welcher Unsicherheit die aktuelle Kalibration behaftet ist, d.h. mit welcher Unsicherheit bei der Verwendung der aktuellen Kalibrierung aus einem Messsignal auf eine Konzentration des Analyten in der Körperflüssigkeit geschlossen werden kann. Insbesondere kann die Kalibrationsqualität mindestens ein Konfidenzintervall umfassen, also beispielsweise eine Standardabweichung oder ein Vielfaches der Standardabweichung.

Auch kann die Änderung der bestimmten Steigung und des Achsenabschnittes im Vergleich zu vorhergehenden Kalibrationen dabei als Prüfgröße verwendet werden, ob der Sensor noch die notwendigen Sensoreigenschaften besitzt. Dadurch werden beispielsweise Ausreißer erkannt, wobei diese als Kalibrationsergebnis verworfen werden, um eine neue Kalibration auszuführen. Weicht die Steigung einer aktuellen Kalibration mehr als ein vorbestimmtes Maß (entsprechend dem Konfidenzintervall) von der Steigung der vorhergehenden oder von den Steigungen der vorhergehenden Kalibrationen ab, so wird die aktuelle Kalibration verworfen und eine neue Kalibration wird ausgeführt (d.h. die Kalibration wird wiederholt).

Das Verfahren kann weiterhin derart durchgeführt werden, dass ein Benutzer zur Durchführung des Kalibrierverfahrens aufgefordert wird, insbesondere automatisch. Dabei können optional verschiedene Zustände unterschieden werden, z.B. eine Empfehlung einer weiteren Aufnahme eines Referenzwertes mithilfe einer konventionellen Blutglukosemessung zur Verbesserung der Kalibration oder die verpflichtende Aufnahme einer Referenzmessung mit der Abschaltung des Systems, wenn diese Kalibration innerhalb eines definierten Zeitraumes nicht durchgeführt wird. Die Aufforderung kann beispielsweise mittels mindestens einer optischen und/oder akustischen und/oder haptischen Anzeige erfolgen, welche den Benutzer zur Durchführung des Kalibrierverfahrens auffordert. Die Aufforderung kann insbesondere automatisch erfolgen, beispielsweise mittels einer Steuerung des Messgeräts.

Der Benutzer kann insbesondere zur Durchführung des Kalibrierverfahrens aufgefordert werden, wenn mindestens eine Kalibrationsbedingung vorliegt, also eine Bedingung, welche die Durchführung eines Kalibrierverfahrens sinnvoll erscheinen lässt. Insbesondere kann diese Bedingung beispielsweise darin bestehen, dass mindestens ein aktuelles Messsignal in einem Bereich liegt, in dem eine Kalibrationsqualität unterhalb einer vorgegebenen Qualitätsschwelle liegt. Dies kann beispielsweise dadurch bedingt sein, dass innerhalb dieses Bereiches noch keine ausreichende Anzahl von Kalibrationspunkten vorliegt, beispielsweise da bisher in diesem Bereich noch keine Messsignale aufgetreten sind. Diese Verfahrensvariante berücksichtigt, dass, im Gegensatz zu Laborversuchen, im praktischen Einsatz an einem Benutzer Analytkonzentrationen für die Kalibration nicht willkürlich vorgegeben werden können, sondern dass die Kalibration mit tatsächlich am Benutzer auftretenden Analytkonzentrationen durchgeführt werden muss. Tritt dann beispielsweise der Fall ein, dass ein bislang noch nicht dagewesener Wert einer Analytkonzentration vorliegt, so kann der Benutzer automatisch darauf hingewiesen werden, dass nun ein günstiger Zeitpunkt zur Durchführung eines Kalibrierverfahrens vorliegt. Wird eine aktuelle Kalibrationsqualität bestimmt, so kann ein Benutzer auch insbesondere darüber informiert werden, wenn ein aktuelles Messsignal in einem Bereich liegt, in dem keine ausreichende Kalibrationsqualität vorliegt. In diesem Fall kann beispielsweise wiederum eine optische, eine akustische oder eine haptische Anzeige bzw. Signalisierung an den Benutzer erfolgen. Diese Information kann auch beinhalten, dass eine Messung der Analytkonzentration automatisch abgebrochen oder unterbrochen wird, gegebenenfalls gemeinsam mit einem Hinweis darauf, dass keine ausreichende Kalibrationsqualität vorliegt, was auch eine Abweichung von einem gültigen Bereich nach oben oder nach unten beinhalten kann.

Wie oben dargelegt, kann das Kalibrierverfahren insbesondere wiederholt durchgeführt werden, beispielsweise in regelmäßigen oder unregelmäßigen Zeitabständen, beispielsweise aufgrund einer Aufforderung durch das Messgerät. Die dabei neu gewonnene Kalibrationsinformation, beispielsweise eine neue wahrscheinliche Steigung, kann dann für die prospektive Auswertung zukünftiger Messungen verwendet werden. Alternativ oder zusätzlich kann jedoch auch eine retrospektive Auswertung bereits durchgeführter Messungen erfolgen, so dass diese Messungen neu bewertet werden können. Bei der wiederholten Durchführung des Kalibrierverfahrens und/oder von Teilen des genannten Kalibrierverfahrens, beispielsweise indem lediglich einzelne neue Kalibrationspunkte neu bestimmt werden, können dann auch ältere Kalibrationspunkte verworfen werden. Wie oben ausgeführt, kann dieses Verwerfen entweder eine vollständige Nichtberücksichtung dieser älteren Kalibrationspunkte beinhalten oder eine zumindest geringere Wichtung dieser älteren Kalibrationspunkte. Beispielsweise können unter älteren Kalibrationspunkten Kalibrationspunkte verstanden werden, welche älter sind als mindestens eine vorgegebene Zeitschwelle.

Spezifische Ausführungsformen des Verfahrens unterscheiden zwischen den Zuständen:

(A) Verpflichtung zur Referenzmessung (d.h. Kalibrierung), "Must Calibrate"
(B) Empfehlung zur Referenzmessung, "Recomend to Calibrate"
(C) Bereitschaft zur Referenzmessung, "May Calibrate" oder "Ready to Calibrate" und
(D) gute Bedingungen zur Referenzmessung, "Nice to Calibrate".
(E) Kalibration aufgrund von schlechten Signaleigenschaften nicht möglich, "Must Not Calibrate"

Im Zustand (A) wird beispielsweise eine Anzeige von Messergebnissen blockiert, um den Nutzer zu einer Kalibrierung zu zwingen. Durch eine erfolgreiche Kalibrierung bzw. Referenzmessung wird dieser Zustand verlassen und eine Messung einschließlich der Anzeige der resultierenden Messergebnis wird ermöglicht, wobei die Messung auf der erfolgreichen Kalibrierung bzw. Referenzmessung basiert. Zustand (A) tritt ein, wenn die zuletzt ausgeführte Kalibrierung / Referenzmessung mehr als eine vorbestimmte Zeitdauer zurückliegt oder wenn keine Kalibrierung / Referenzmessung mit dem vorliegenden Messsystem bislang stattfand. Zustand (A) kann ferner eintreten, wenn das Messsignal in einem Bereich liegt, der außerhalb eines vorbestimmten, üblichen Messbereichs liegt und auf einen Fehler hindeutet, beispielsweise ein Kontaktfehler.Zustand (A) tritt nicht auf, wenn die Messwerte unter einer vorbestimmten Grenze liegen, wobei die Grenze auf einen kritischen Zustand des Messobjekts hinweist, beispielsweise Hypoglykämie. Bei Messwerten, die unterhalb dieser Grenze liegen, wird zwingend ein anderer Zustand aufgerufen, in dem Messungen durchgeführt und angezeigt werden, bevorzugt Zustand (B) oder auch die Zustände (C) und (D). In den Zuständen (B), (C) und (D) (sowie (E)) können Messungen ausgeführt und deren Ergebnisse angezeigt werden. Zustand (A) kann ferner abgebrochen werden, wenn länger als eine vorbestimmte Länge andauert, vorzugsweise wird dann die Messung abgebrochen.

Im Zustand (B) wird eine Aufforderung zur Kalibrierung angezeigt bzw. das Erfordernis einer Kalibrierung / Referenzmessung dem Nutzer mitgeteilt. Zustand (B) tritt ein, wenn ein Messsignal außerhalb des Kalibrierungsbereichs liegt, d.h. außerhalb von den Referenzpunkten der Referenzmessung. Vorzugsweise tritt Zustand (B) ein, wenn das Messsignal außerhalb eines Bereichs liegt, der sich durch den Kalibrierungsbereich, einen sich daran anschließenden, unteren Toleranzbereich und einen daran anschließenden, oberen Toleranzbereich ergibt. Der obere Toleranzbereich, der sich in Richtung der höheren Werte an den Kalibrierungsbereich anschließt (d.h. an den größten Wert der Referenzmessungen), hat eine Breite, die einem vorgegebenen Anteilsfaktor multipliziert mit der oberen Grenze des Kalibrierungsbereichs oder multipliziert mit dem Messsignal entspricht. Die Breite des unteren Toleranzbereichs ist vorzugsweise durch einen Absolutwert vorgegeben. Schließlich kann in Zustand (B) eingetreten werden, wenn eine vorgegebene Zeitdauer seit dem letzten Kalibrieren verstrichen ist. Diese Zeitdauer ist kürzer als die Zeitdauer, die im Rahmen von Zustand (A) verwendet wird. Zustand (B) entspricht einer noch gültigen Kalibrierung, deren Genauigkeit jedoch gering ist, weshalb eine Kalibrierung mit höherer Präzision ausgeführt werden sollte.

Zustand (C) entspricht einem Standardzustand, der die allgemeine Bereitschaft der Vorrichtung zur Kalibration darstellt, beispielsweise wenn bereits eine Kalibration durchgeführt wurde. Zustand (C) entspricht einem Zustand, in dem eine Kalibration nicht notwendigerweise zu einer Verbesserung der Messgenauigkeit führt, im Gegensatz zu Zuständen (A) und (B).

In Zustand (D) wird eingetreten, wenn die Bedingungen für eine präzise Kalibrierung Referenzmessung besonders gut sind. Dies ist erfüllt, wenn die Messergebnisse (über eine vorbestimmte Zeitdauer) weniger als eine vorgegebene Schwankungsbreite schwanken. Mit anderen Worten ist dies erfüllt, wenn die Dynamik der Messergebnisse unter einem bestimmten Schwellwert liegt. Die geringe Dynamik bzw. Streuung deutet auf einen stabilen Zustand bzw. einer hohen Signifikanz der Referenzmessung hin, wobei dies wiederum mit hoher Genauigkeit verknüpft ist. In Zustand (D) wird ein Hinweis auf gute Kalibrationsbedingungen bzw. auf die Möglichkeit der Verbesserung der Messpräzision angezeigt.

In Zustand (E) wird eingetreten, wenn die Signaleigenschaften auf eine hohe Streuung oder eine starke, momentane Änderung des Signals (d.h. der Messwerte) schließen lassen, die über einer vorbestimmten Maximalvarianz bzw Maximaländerung liegt. In Zustand (E) würde aufgrund der Streuung bzw. aufgrund des starken Trends (starker Messsignalanstieg oder -abfall bezogen auf die betreffende Zeitdauer), die bislang geltende Kalibration Referenzmessung durch einen unpräziseren Wert ersetzt werden, da die starke Streuung / der starke Anstieg mit einem großen Fehler verknüpft ist. In Zustand (E) wird angezeigt, dass keine Kalibration ausgeführt werden soll. Ferner werden Befehle zur Kalibration, sofern sie trotzdem eingeben werden, in Zustand (E) ignoriert und die vorhergehende Referenzmessung / Kalibration wird beibehalten. In Zustand (E) können Messungen durchgeführt werden und das Messergebnis wird angezeigt. Optional kann angezeigt werden, dass die Messung wiederholt werden sollte (insbesondere nach einer Kalibration außerhalb des Zustands (E)), bzw. dass das Messergebnis keine hohe Präzision aufweist. Bei der Messung wird die aktuelle Kalibration verwendet, wobei ein Kalibrationsbefehl, der in Zustand (E) eingeben wird, die aktuelle Kalibration unverändert lässt. In Zustand (E) wird eine Kalibration verwendet, die vor dem Eintritt in Zustand (E) ausgeführt wurde (d.h. deren Referenzmessung vor Eintritt in Zustand (E) ausgeführt wurde).

Bevorzugt werden Zustände, die auf eine besonders hohe Dynamik oder Streuung der hinweisen (bsp. Zustand (E)), und Zustände die auf eine besonders geringe Dynamik oder Streuung hinweisen (bsp. Zustand (D)), erkannt, indem das zeitliche bzw. stochastische Verhalten des Messsignal selbst betrachtet wird. Hierbei wird entweder ein Wert berechnet, der einer Varianz bzw. Streuung entspricht, oder ein Wert berechnet, der die erste zeitliche Ableitung wiedergibt, oder beides. Dieser Wert (diese Werte) dienen gegenüber entsprechenden Grenzwerten als Entscheidungsgrundlage, ob eine besonders hohe / niedrige Dynamik oder Streuung vorliegt. Die Werte können insbesondere das Maximum der Dynamik (d.h. der ersten Ableitung) bzw. der Streuung des Messsignals wiedergeben. Die Betrachtung des Messsignals betrifft vorzugsweise ein Zeitfenster des Messginals oder die letzten N Werte des Messsignals, wobei N eine natürliche Zahl > 0 (vorzugsweise > 10, > 100 oder > 1000) ist.

Vorzugsweise sieht das Verfahren vor, neben einem Start- oder Endzustand, im Wesentlichen nur zwischen den Zuständen (A) - (E) zu wechseln.

Weiterhin kann das Verfahren auch derart modifiziert werden, dass die Kalibrationspunkte und/oder Bestandteile der Kalibrationspunkte, beispielsweise die Messsignale und/oder die Referenzwerte, einzeln oder gemeinsam einem Glättungsverfahren unterzogen werden. Alternativ oder zusätzlich zu der Anwendung eines Glättungsverfahrens bei dem Kalibrierverfahren kann auch die mindestens eine Messung unter Durchführung eines oder mehrerer Glättungsverfahren erfolgen. Die Kalibrationspunkte und/oder die Messsignale können beispielsweise mindestens einem Glättungsverfahren unterzogen werden, insbesondere einem Glättungsverfahren mittels mindestens eines Exponentialfilters. Ausführungsbeispiele derartiger Glättungsverfahren werden unten noch näher erläutert.

Weiterhin kann das Verfahren derart weitergebildet werden, dass die Kalibrationspunkte und/oder Bestandteile dieser Kalibrationspunkte und/oder die Messsignale mindestens einem Linearisierungsschritt unterworfen werden. In diesem Linearisierungsschritt kann ein zumindest näherungsweise linearer Zusammenhang zwischen den Messwerten und den Referenzwerten hergestellt werden. Dieses Verfahren bietet sich insbesondere dann an, wenn ein nicht-linearer Zusammenhang zwischen den Analytkonzentrationen und den Messsignalen festgestellt wird oder bekannt ist. Beispielsweise kann der Signalverlauf der Messsignale ein Sättigungsverhalten zeigen, also insbesondere bei höheren Werten der Messsignale nach unten von einem linearen Verlauf abweichen. In diesem Fall kann vor Auswertung der Kalibrationspunkte und/oder vor Auswertung der Messsignale der genannte Linearisierungsschritt vorgenommen werden. Dabei können die Kalibrationspunkte und/oder die Messsignale beispielsweise einer Transformation unterworfen werden, welche wiederum bekanntermaßen einen linearen Verlauf herstellt. Bei Sättigungsverhalten kann dies beispielsweise unter Verwendung mindestens eines Logarithmus erfolgen, insbesondere bei Messwerten in einem vorgegebenen Sättigungsbereich, in dem von einer teilweisen Sättigung des Messsystems ausgegangen werden kann. Beispiele für Linearisierungsschritte sind im Vorangehenden dargestellt und werden unten noch näher ausgeführt.

Weiterhin wird ein Verfahren zum Betrieb eines Messgeräts zur Erfassung mindestens eines Analyten einer Körperflüssigkeit vorgeschlagen, welches eine Datenreduktion umfasst. Dieses Verfahren wird grundsätzlich als Weiterbildung des oben beschriebenen Verfahrens in einer oder mehreren der dargestellten Verfahrensvarianten ausgestaltet, so dass die im Folgenden beschriebenen Merkmale als zusätzliche Merkmale anzusehen sind.

Bei diesem zweiten Aspekt eines Verfahrens zum Betrieb eines Messgeräts zur Erfassung mindestens eines Analyten in einer Körperflüssigkeit wird bei einer Messung eine Mehrzahl von Messsignalen des Messgeräts über einen Messzeitraum erfasst. Beispielsweise kann dieser Messzeitraum einen typischen Messzeitraum eines kontinuierlich messenden Blutglucose-Sensors umfassen, beispielsweise im Minutenbereich, in welcher beispielsweise jeweils im Subminutenabstand Messsignale erfasst werden.

Weiterhin wird mindestens eine Datenreduktion durchgeführt. Bei dieser Datenreduktion wird die Mehrzahl der Messwertpaare aus Messsignal und Messzeitpunkt auf ein oder wenige Messwertpaare reduziert. Dabei können eines oder mehrere der folgenden Datenreduktionsverfahren angewandt werden.

In einem ersten möglichen Datenreduktionsverfahren wird mittels mindestens eines robusten Schätzverfahrens aus der Mehrzahl von Messsignalen mindestens ein wahrscheinliches Messsignal für den Messzeitraum bestimmt. Die Mehrzahl der Messsignale wird dann durch das wahrscheinliche Messsignal ersetzt. Bezüglich des robusten Schätzverfahrens kann auf die obige Beschreibung robuster Schätzverfahren verwiesen werden. Wiederum kann auch eine Kombination robuster Schätzverfahren eingesetzt werden. Besonders bevorzugt ist die Verwendung eines Medians. Alternativ oder zusätzlich könnten jedoch allgemein beispielsweise auch andere Algorithmen eingesetzt werden, welche auf einem oder mehreren Permutationsalgorithmen und/oder einem oder mehreren Sortieralgorithmen basieren, aus denen die Bildung eines Medians lediglich ein bevorzugtes Beispiel darstellt.

Alternativ oder zusätzlich kann das Datenreduktionsverfahren auch derart durchgeführt werden, dass die Messsignale mit den zugehörigen Messzeitpunkten zu Messwertpaaren zusammengefasst werden. Die Messwertpaare können darüber hinaus zusätzliche Informationen umfassen. Weiterhin wird eine Mehrzahl möglicher Steigungen zwischen den Messwertpaaren ermittelt, wobei mittels mindestens eines robusten Schätzverfahrens aus der Mehrzahl möglicher Steigungen eine wahrscheinliche Steigung ermittelt wird. Bezüglich des robusten Schätzverfahrens gilt wiederum das oben Gesagte analog. Insbesondere kann dieses robuste Schätzverfahren wiederum unter Verwendung mindestens eines Medians durchgeführt werden. Alternativ oder zusätzlich könnten jedoch allgemein beispielsweise auch andere Algorithmen eingesetzt werden, welche auf einem oder mehreren Permutationsalgorithmen und/oder einem oder mehreren Sortieralgorithmen basieren, aus denen die Bildung eines Medians lediglich ein bevorzugtes Beispiel darstellt.

Aus der Mehrzahl möglicher Steigungen wird dann eine Mehrzahl von Geraden durch die Messwertpaare gebildet. Dies kann beispielsweise dadurch erfolgen, dass durch jeden einem Messwertpaar entsprechenden Punkt oder zumindest durch eine Mehrzahl dieser Punkte Geraden gebildet werden, welche die wahrscheinliche Steigung aufweisen, so dass eine Schar paralleler Geraden durch die Punkte entsteht. Weiterhin wird mindestens ein repräsentativer Zeitpunkt für den Messzeitraum gewählt, beispielsweise die Hälfte des Zeitintervalls des Messzeitraums. Grundsätzlich kann der repräsentative Zeitpunkt jedoch beliebig innerhalb des Messzeitraums gewählt werden, beispielsweise der Anfangspunkt oder der Endpunkt des Messzeitraums oder ein beliebiger Zwischenpunkt. Anschließend werden die Funktionswerte der genannten Geraden, also aller oder mehrerer der Geraden, für den repräsentativen Zeitpunkt ermittelt, wobei aus den Funktionswerten mittels mindestens eines robusten Schätzverfahrens mindestens ein wahrscheinlicher Funktionswert ermittelt wird. Dann wird die Mehrzahl der Messwertpaare durch mindestens ein repräsentatives Messwertpaar ersetzt, welches den mindestens einen wahrscheinlichen Funktionswert und den mindestens einen repräsentativen Zeitpunkt umfasst. Das robuste Schätzverfahren kann wiederum insbesondere unter Verwendung mindestens einer Bildung eines Medians durchgeführt werden. Alternativ oder zusätzlich könnten jedoch allgemein beispielsweise auch andere Algorithmen eingesetzt werden, welche auf einem oder mehreren Permutationsalgorithmen und/oder einem oder mehreren Sortieralgorithmen basieren, aus denen die Bildung eines Medians lediglich ein bevorzugtes Beispiel darstellt. Beispiele des beschriebenen Datenreduktionsverfahrens werden unten näher ausgeführt.

Bei der Datenreduktion in einer oder beiden der beschriebenen Varianten kann weiterhin aus der Mehrzahl von Messsignalen mindestens ein Streumaß bestimmt werden, insbesondere eine Standardabweichung und/oder ein Quantilsmaß.

Weiterhin ist es bei den Datenreduktionsverfahren, insbesondere der zweiten Variante des Datenreduktionsverfahrens, möglich, aus der Mehrzahl von Messsignalen einen Trend zu bestimmen. Dieser Trend, welcher einen zeitlichen Verlauf des Messsignals auf kontinuierlicher oder ordinaler Skala vorhersagt, kann insbesondere aus der wahrscheinlichen Steigung ermittelt werden, welche für sich genommen bereits einen derartigen Trend angibt. Als ein Beispiel können sekündliche Messpunkte durch Mittelung (ggf. unter Ausschluss von Ausreißern) zu einem einzigen, minütlichen Messpunkt zusammengefasst werden. Weiterhin kann bei dem Verfahren mindestens eine Aussage über eine Signalqualität der Mehrzahl von Messsignalen getroffen werden. Diese Aussage über die Signalqualität kann insbesondere durch einen Vergleich zwischen robusten Schätzern der Messsignale, beispielsweise wiederum des Medians (und/oder eines anderen Permutationsalgorithmus und/oder Sortieralgorithmus) und/oder der Quantilsmaße, und parametrischen Schätzern der Messsignale, beispielsweise des Mittelwerts und/oder der Standardabweichung, erfolgen.

In einem dritten Aspekt der vorliegenden Erfindung wird wiederum ein Verfahren zum Betrieb eines Messgeräts zur Erfassung mindestens eines Analyten in einer Körperflüssigkeit vorgeschlagen. Das Verfahren weist wiederum zusätzlich die Merkmale der oben beschriebenen Verfahrensvarianten gemäß dem ersten und/oder dem zweiten Aspekt der Erfindung auf, so dass für mögliche weitere Ausgestaltungen des im Folgenden beschriebenen dritten Aspekts auf die obige Beschreibung verwiesen werden kann.

Bei dem dritten Aspekt der Erfindung wird in mindestens einem Kalibrierverfahren eine Kalibrierung von Messsignalen des Messgeräts gegen Referenzwerte zugehöriger Referenzmessungen durchgeführt. Dabei kann es sich um eine oder mehrere Referenzmessungen handeln. Bezüglich möglicher Ausgestaltungen der Referenzmessungen kann auf die obige Beschreibung verwiesen werden. Weiterhin wird bei dem dritten Aspekt der Erfindung in dem Verfahren eine Kalibrationsstrategie angewandt. Unter einer Kalibrationsstrategie wird allgemein eine Strategie zur Optimierung der Kalibration, beispielsweise zur stetigen Verbesserung der Genauigkeit der Kalibration, beispielsweise einer Kalibrationsqualität, verstanden.

Die Kalibrationsstrategie kann eine oder mehrere der im Folgenden beschriebenen Verfahrensschritte umfassen.

So kann in Abhängigkeit von einer aktuellen Kalibrationsqualität, die sich zum Beispiel aus einer Plausibilitätsbetrachtung ergibt, ein für eine Messung verwendbarer Bereich von Messsignalen des Messbereichs eingeschränkt werden. So kann beispielsweise als verwendbarer Bereich ein Bereich bestimmt werden, welche durch die bisherige Kalibration mit ausreichender Kalibrationsqualität abgedeckt ist. Beispielsweise kann es sich dabei um einen Bereich handeln, innerhalb dessen Referenzwerte vorliegen, optional zuzüglich eines oder mehrerer Intervalle oberhalb oder unterhalb dieses Bereichs, beispielsweise eines vorgegebenen Prozentsatzes. Insbesondere kann bei einer Erfassung von Messsignalen außerhalb eines verwendbaren Bereichs ein Hinweis an einen Benutzer erfolgen, beispielsweise ein Hinweis, dass eine Untergrenze überschritten ist, oder dass eine Obergrenze überschritten ist. Dies kann beispielsweise wiederum durch eine optische, eine akustische oder eine haptische Anzeige erfolgen.

Alternativ oder zusätzlich kann die Kalibrationsstrategie auch eine Aufforderung an einen Benutzer zur erneuten Durchführung des Kalibrierverfahrens umfassen. Beispielsweise kann diese Aufforderung wiederum über ein Anzeigenelement erfolgen, beispielsweise wenn die bisherige Kalibrationsqualität nicht ausreichend ist und/oder wenn ein günstiger Zeitpunkt zur Durchführung eines Kalibrierverfahrens vorliegt.

Wiederum alternativ oder zusätzlich kann die Kalibrationsstrategie auch einen Verfahrensschritt umfassen, bei welchem ein Benutzer darüber informiert wird, dass ein günstiger Zeitpunkt zur Durchführung des Kalibrierverfahrens vorliegt. Insbesondere kann diese Information wiederum über ein Anzeigenelement erfolgen. Ein günstiger Zeitpunkt kann, wie oben bereits ausgeführt, insbesondere bei einer aktuell geringen Signaldynamik der Messsignale und/oder bei einer zu erwartenden starken Abweichung der Konzentration des mindestens einen Analyten in der Körperflüssigkeit von bisherigen Analytkonzentrationen vorliegen.

Wiederum alternativ oder zusätzlich kann die Kalibrationsstrategie auch einen Verfahrensschritt umfassen, bei welchem ein Benutzer in regelmäßigen oder unregelmäßigen Abständen, beispielsweise in vorgegebenen Abständen, zur Durchführung oder erneuten Durchführung des Kalibrationsverfahrens aufgefordert wird. Beispielsweise könnte eine derartige Aufforderung nach einer vorgegebenen Anzahl an Stunden seit der letzten Durchführung des Kalibrationsverfahrens erfolgen.

Neben dem Verfahren in einer oder mehreren der oben vorgeschlagenen Ausgestaltungen wird weiterhin ein Computerprogramm mit Programmcode zur Durchführung des Verfahrens in einer oder mehreren der vorgeschlagenen Varianten, wenn das Programm in einem Computer ausgeführt wird, vorgeschlagen. Insbesondere kann das Computerprogramm in einem Computer einer Steuerung des Messgeräts zur Erfassung mindestens eines Analyten in einer Körperflüssigkeit durchgeführt werden. Das Computerprogramm kann insbesondere auf einem Computer-lesbaren Datenträger gespeichert sein.

Neben dem Verfahren und dem Computerprogramm wird weiterhin ein Messgerät zur Erfassung mindestens eines Analyten in einer Körperflüssigkeit vorgeschlagen. Das Messgerät kann insbesondere mindestens einen kontinuierlich messenden Sensor, insbesondere einen kontinuierlich messenden Blutglukose-Sensor, umfassen. Das Messgerät ist eingerichtet, um mindestens ein Messsignal entsprechend einer Konzentration des Analyten in der Körperflüssigkeit zu erzeugen. Das Messgerät ist weiterhin eingerichtet, um mittels einer unabhängigen Referenzmessung erfasste Referenzwerte zu empfangen. Das Messgerät weist mindestens eine Steuerung auf, insbesondere eine Steuerung mit mindestens einem Computer, wobei die Steuerung eingerichtet ist, um ein Verfahren in einer oder mehreren der oben beschriebenen Verfahrensvarianten durchzuführen. Das Messgerät kann dabei eine oder mehrere Komponenten umfassen, welche zusammenhängend oder auch nicht-zusammenhängend ausgestaltet sein können. So kann das Messgerät beispielsweise mehrere einzelne Messgeräte umfassen, also dezentral ausgestaltet sein, wobei die einzelnen Messgeräte im Sinne des erfindungsgemäßen Messgeräts zusammenwirken. Beispielsweise können dies der kontinuierlich messende Sensor und ein Steuergerät und/oder ein weiteres Messgerät sein, welche beispielsweise drahtgebunden oder drahtlos mit einander in Verbindung stehen können. Dementsprechend kann unter einem Messgerät auch ein Messsystem mit mehreren einzelnen Komponenten, vorzugsweise mehreren miteinander in einer kommunikativen Verbindung stehenden Komponenten, verstanden werden. Alternativ ist jedoch auch eine Integration in ein einziges Messgerät möglich.

Das vorgeschlagene Verfahren, das Computerprogramm und das Messgerät in einer oder mehreren der oben beschriebenen Ausführungsvarianten weisen gegenüber bekannten Verfahren und Vorrichtungen dieser Art zahlreiche Vorteile auf. So lässt sich insbesondere eine stabile Kalibration erreichen, die auch gegenüber Ausreißern vergleichsweise unempfindlich ist. Im Unterschied beispielsweise zu dem in US 2006/0281985 beschriebenen Verfahren, in welchem Medianberechnungen lediglich bei Messwerten eingesetzt werden, wird erfindungsgemäß gemäß dem ersten Aspekt der vorliegenden Erfindung direkt eine Regression für das Kalibrierverfahren eingesetzt. Die wahrscheinliche Steigung, welche dabei ermittelt wird, spiegelt die Sensitivität des Messgeräts, beispielsweise des Messsystems, wider. Das Verfahren ist auf einfache und schnelle Weise implementierbar und ist insbesondere für kontinuierlich messende Blutglukose-Messgeräte, in welchen die oben beschriebenen Schwierigkeiten bei der Kalibrierung auftreten können, geeignet.

Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:

Figuren 1A-1D    Verfahrensschritte eines ersten Ausführungsbeispiels eines erfindungsgemäßen Verfahrens mit einem Kalibrierverfahren zur Bestimmung einer wahrscheinlichen Steigung und optional einem wahrscheinlichen Achsenabschnitt;

Figur 2    ein Ausführungsbeispiel einer Durchführung einer Plausibilitätsbetrachtung;

Figur 3    ein weiteres Ausführungsbeispiel einer Plausibilitätsbetrachtung unter Verwendung eines Failsafe-Mechanismus;

Figur 4    ein schematisches Ablaufdiagramm eines Ausführungsbeispiels eines erfindungsgemäßen Verfahrens; und

Figur 5    ein Ausführungsbeispiel eines erfindungsgemäßen Messgeräts.

Ausführungsbeispiele

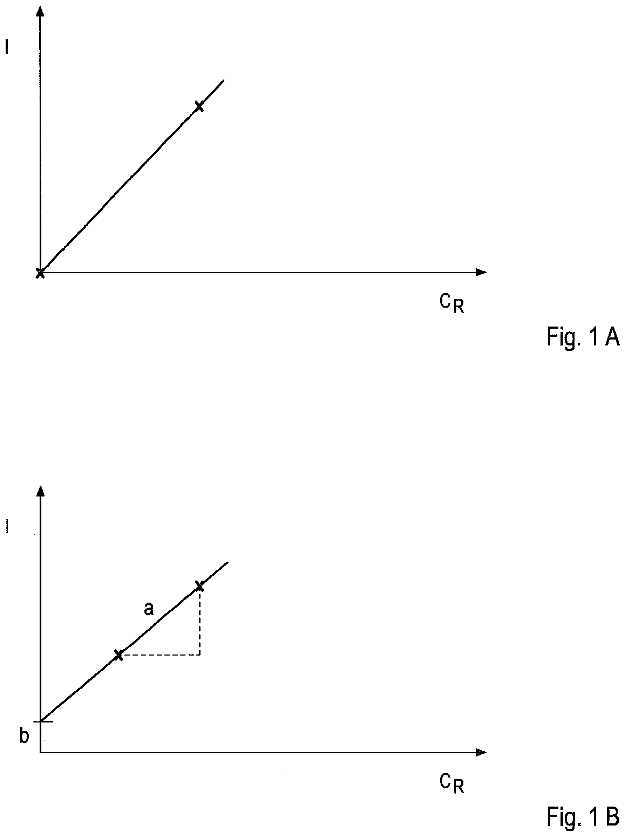

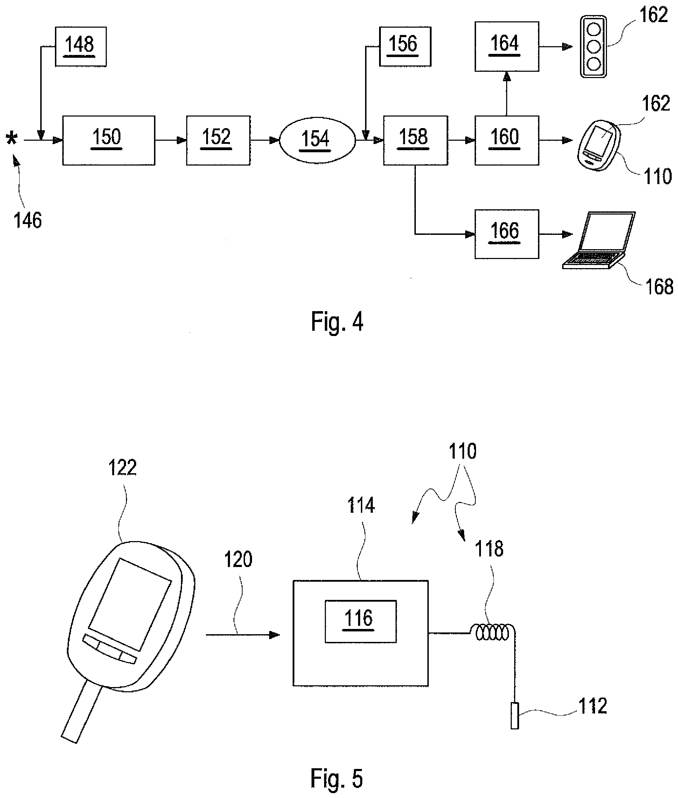

Im Folgenden sollen anhand der Figuren 1A bis 5 Ausführungsbeispiele eines erfindungsgemäßen Verfahrens sowie eines erfindungsgemäßen Messgeräts 110 zur Erfassung mindestens eines Analyten in einer Körperflüssigkeit dargestellt werden. In Figur 5 ist ein Ausführungsbeispiel eines derartigen Messgeräts 110 schematisch dargestellt. In diesem Fall handelt es sich bei dem Messgerät um ein Messgerät zur kontinuierlichen Blutglukoseüberwachung mittels eines kontinuierlich messenden Blutglukosesensors 112. Beispielsweise kann dieser kontinuierlich messende Sensor in ein interstitielles Gewebe eines Benutzers implantiert werden und dort über einen Zeitraum von beispielsweise einigen Tagen verbleiben. Das Messgerät 110 umfasst weiterhin eine Steuerung 114 mit mindestens einem Computer 116. Weiterhin kann das Messgerät 110 ein oder mehrere Anzeigenelemente und/oder ein oder mehrere Bedienelemente umfassen sowie gegebenenfalls eine oder mehrere Schnittstellen, um eine Interaktion eines Benutzers und/oder eines anderen Geräts, beispielsweise eines Computers oder Computernetzwerks, mit dem Messgerät 110 zu ermöglichen. Die Steuerung 114 ist, wie in Figur 5 durch die Bezugsziffer 118 angedeutet, drahtlos oder drahtgebunden mit dem kontinuierlich messenden Blutglukose-Sensor 112 verbunden.

Weiterhin ist das Messgerät 110 eingerichtet, um einen oder mehrere unabhängig erfasste Referenzwerte zu empfangen. Dieser Empfang von Referenzwerten ist mit der Bezugsziffer 120 gekennzeichnet. Zur Erzeugung der Referenzwerte kann ein separates Referenzmessgerät 122 vorgesehen sein, beispielsweise ein Teststreifen-Messgerät zur Spot-Messung von Blutglukosewerten in Blutstropfen. Alternativ oder zusätzlich kann das Referenzmessgerät 122 auch ganz oder teilweise in das Messgerät 110 selbst integriert sein. Auch dies soll vom Begriff des Empfangens von Referenzwerten umfasst sein.

In den Figuren 1A bis 1D ist ein Ausführungsbeispiel eines Kalibrierverfahrens zur prospektiven Kalibrierung des Messgeräts 110 dargestellt. Für das Kalibrationsverfahren wird eine zumindest näherungsweise lineare Beziehung zwischen Messsignalen des Messgeräts und Referenzwerten aus Referenzmessungen angenommen. Alternativ kann bei einer nichtlinearen Beziehung durch eine fest vorgegebene, nichtlineare Transformation eine lineare Beziehung (d.h. ein linearer Verlauf der Messwert-Konzentrationslinie) hergestellt werden, insbesondere als vorgelagerter Linearisierungsschritt bzw. Linearisierungstransformation der Messwerte bzw. des Messsignals, bevor diese bzw. dieses erfindungsgemäß anhand der Steigungen betrachtet werden bzw. wird. Insbesondere können die oben dargestellten Linearisierungstransformation angewandt werden. Die Messsignale des Messgeräts sind dabei in den Figuren 1A-1D mit I bezeichnet und sind exemplarisch angegeben in Ampere, Grundsätzlich sind jedoch beliebige Einheiten und/oder Größen als Messsignale möglich. Die Messsignale I sind dabei jeweils auf der y-Achse in den Figuren 1A-1D aufgetragen. Die Referenzwerte sind hingegen in den Figuren 1A-1D mit $c_R$ bezeichnet und sind in beliebigen Konzentrationseinheiten angegeben. Beispielsweise kann es sich dabei um Referenzwerte handeln, welche mittels eines Referenzmessgeräts 122 in Form eines Handgeräts, beispielsweise analog zu Figur 5, ermittelt wurden. Alternativ oder zusätzlich können diese Referenzwerte jedoch auch, wie oben dargestellt, vom Messgerät 110 selbst mittels eines Referenzmessverfahrens ermittelt werden, beispielsweise indem ein derartiges Referenzmessgerät 122 in das Messgerät 110 integriert ist. Der oben beschriebene lineare Zusammenhang zwischen den Messsignalen des Messgeräts und den Referenzwerten wird zumindest in einem Messbereich angenommen, beispielsweise innerhalb eines üblicherweise nicht unter- bzw. überschrittenen Messbereichs.

Ziel des Kalibrierverfahrens ist es, den genannten Zusammenhang zu bestimmen. Ein linearer Zusammenhang ist definiert durch eine Steigung a und einen Achsenabschnitt b einer Geradenfunktion mit der Funktionsgleichung I= $a*c_R$ + b. Um später bei einer eigentlichen Messung aus einem Messsignal des Messgeräts 110 auf eine Analytkonzentration c, beispielsweise eine Blutglukosekonzentration, ist die oben genannte Geradengleichung zu invertieren, so dass die Gleichung c= (1/a)*I - b/a anwendbar ist. Die Parameter a und b bestimmen also eine Kalibrierung des Messgeräts 110.

Bei der prospektiven Kalibrierung werden also, optional iterativ, Kalibrationspunkte bestimmt, welche jeweils ein Messsignal des Messgeräts 110 und mindestens einen Referenzwert umfassen. Aus diesen Kalibrationspunkten werden die Steigung a und der Achsenabschnitt b berechnet, beispielsweise nach dem in der oben genannten Veröffentlichung von H. Passing und W. Bablok beschriebenen Verfahren.

Um eine möglichst hohe Qualität der Parameterberechnung zu gewährleisten, wird in einer bevorzugten Ausführungsform der Erfindung die Auswahl der Referenzwerte durch das Referenzmessgerät gesteuert. Insbesondere wird ein Referenzwert nicht zur Kalibration verwendet (d.h. ausgeschlossen), wenn das zugehörige Signal des Messgerätes eine schlechte Qualität (hohe Streuung der Messwerte) oder eine hohe Dynamik (Trend) aufweist. Dagegen wird der Anwender zu einer Kalibration aufgefordert, wenn das mit den bisher ermittelten Kalibrationsparametern umgerechnete Signal den durch die bereits aufgenommenen Kalibrationswerte überdeckten Bereich verläßt. Dieser Bereich bzw. Toleranzbereich kann z. B. gegeben sein über das Intervall $[c_{lb};c_{ub}]$ mit

$$c_{lb} := \min\{bG_{min} - Cp * d; bG_{min} * (1-d)\} \text{ und } c_{ub} := \max\{bG_{max} + Cp * d; bG_{max} * (1+d)\}$$

Dabei ist Cp ein vorgegebener, charakteristischer Messpunkt, d eine vorgegebene Toleranz und $bG_{min}$ und $bG_{max}$ der minimale und der maximale aufgenommene Referenzwert. Die hier verwendete Toleranz d ist relativ und erwirkt eine Toleranzbreite, die von dem jeweiligen Referenzwert bestimmt wird. Alternativ kann die untere Grenze $c_{lb}$ bestimmt werden durch den minimalen Referenzwert abzüglich einem vorgegebenen, absoluten Toleranzbreitenwert. Tritt ein Messwert unterhalb des Intervalls auf, so wird weder eine Messung blockiert noch eine Anzeige des Messergebnisses. Stattdessen wird das Messergebnis angezeigt und zur Kalibration bzw. zur wiederholten Messung aufgefordert. Insbesondere wird dies in Fällen ausgeführt, in denen ein Messwert einer Konzentration entspricht, die typischerweise als hypoglykämisch angesehen wird (sofern als Analyt Blutzucker erfasst wird). Dadurch wird vermieden, dass eine hypoglykämische Messung nicht ausgeführt bzw. deren Ergebnisse aus dem Grund nicht angezeigt werden, dass die Kalibrationsstategie für diesen Fall (außerhalb des Toleranzbereichs, d.h. außerhalb des kalibrierten Bereichs) eine weitere Kalibrierung zwingend vorsieht (vergleiche Zustand (A)), bevor ein Messwert ausgegeben wird.

In Figur 1A ist ein erster Schritt gezeigt, bei welchem lediglich ein einzelner Kalibrationspunkt vorliegt. Als weiterer Kalibrationspunkt kann beispielsweise der Nullpunkt angenommen werden. Durch diese beiden Punkte, also einen gemessenen Kalibrationspunkt und einen angenommenen Kalibrationspunkt, welcher beispielsweise der Nullpunkt sein kann, wird eine Gerade definiert, deren Steigung und Achsenabschnitt (bei Wahl des Nullpunkts als gewählter Kalibrationspunkt ist dieser naturgemäß gleich 0) bestimmt werden können.

In Figur 1B sind bereits zwei Kalibrationspunkte bekannt, welche eine Gerade definieren. Die Steigung a und der Achsenabschnitt b dieser Geraden können ebenfalls bestimmt werden.

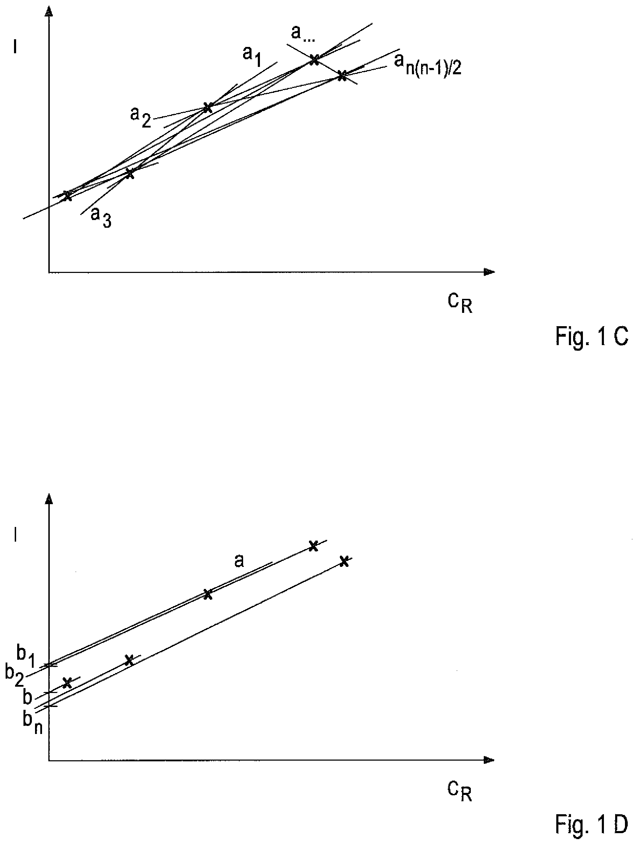

In Figur 1C ist das Verfahren schließlich dargestellt, wenn eine Vielzahl von Kalibrationspunkten bekannt ist, in diesem Fall n Kalibrationspunkte. Zwischen allen diesen Kalibrationspunkten oder einer Mehrzahl dieser Kalibrationspunkte können dann, wie in Figur 1C dargestellt, Geraden gebildet werden, deren Steigungen $a_i$ (mit $i=1, ...,n$) bestimmt werden können. Bei n Kalibrationspunkten liegt damit maximal eine Anzahl von $n*(n-1)/2$ möglichen Steigungen a vor.

Bei dem vorgeschlagenen Verfahren wird dann aus allen oder zumindest mehreren möglichen Steigungen $a_1$ bis maximal $a_{n*(n-1)/2}$ der Median gebildet, welcher als wahrscheinliche Steigung a bezeichnet wird, beispielsweise a= median$\{a_1, a_2,..., a_{n*(n-1)/2}\}$. Alternativ oder zusätzlich könnten jedoch, wie oben bereits dargestellt, allgemein beispielsweise auch andere Algorithmen eingesetzt werden, welche auf einem oder mehreren Permutationsalgorithmen und/oder einem oder mehreren Sortieralgorithmen basieren, aus denen die Bildung eines Medians lediglich ein bevorzugtes Beispiel darstellt. Alternativ oder zusätzlich sind jedoch auch andere robuste Schätzverfahren grundsätzlich einsetzbar.

Die so ermittelte wahrscheinliche Steigung a kann bereits bei nachfolgenden Messungen verwendet werden, beispielsweise indem eine Ursprungsgerade mit dem Inversen dieser Steigung für die Umrechnung eines Messsignals des Messgeräts 110 in eine Blutglukosekonzentration verwendet wird. Besonders bevorzugt ist es jedoch, wenn zusätzlich auch ein Achsenabschnitt b ermittelt wird, welcher einen Offset beschreibt. Dies ist in Figur 1D als optionaler Verfahrensschritt dargestellt. Bei diesem Verfahrensschritt wird durch jeden der Kalibrationspunkte oder zumindest durch eine Mehrzahl der Kalibrationspunkte eine Gerade mit der wahrscheinlichen Steigung gelegt. Der Achsenabschnitt $b_i$ dieser Geraden wird bestimmt, insbesondere berechnet nach der Gleichung $b_i =y_i - a*x_i$. Dabei bezeichnen $x_i$ und $y_i$ die Referenzmessung bzw. das Messsignal des iten Kalibrationspunktes. Aus dieser Mehrzahl von Achsenabschnitten $b_1,..., b_n$, also aus mehreren oder allen dieser Achsenabschnitte, kann dann wiederum durch ein Schätzverfahren, vorzugsweise ein robustes Schätzverfahren, insbesondere ein robustes Schätzverfahren unter Verwendung eines Medians, ein wahrscheinlicher Achsenabschnitt b bestimmt werden, beispielsweise nach der Gleichung b= median$\{b_1, b_2,..., b_n\}$.

Mit der wahrscheinlichen Steigung a und dem wahrscheinlichen Achsenabschnitt b ist der lineare Zusammenhang zwischen den Referenzwerten und den Messsignalen vollständig bekannt. Um bei einer nachfolgenden Messung aus einem Messsignal auf eine Konzentration des Analyten in der Körperflüssigkeit zu schließen, ist dieser lineare Zusammenhang lediglich zu invertieren, beispielsweise gemäß der oben beschriebenen Gleichung $c=(1/a)* I - (b/a)$.

Das anhand der Figuren 1A bis 1D beschriebene Verfahren mit dem Kalibrierverfahren kann auch wiederholt durchgeführt werden. Insbesondere kann das Verfahren derart durchgeführt werden, dass mit jedem neu hinzugekommenen Kalibrationspunkt mit einem Messwertpaar eine Regression gemäß der oben genannten Veröffentlichung von H. Passing und W. Bablok durchgeführt wird. Dies bezüglich kann beispielsweise auf die genannte Veröffentlichung verwiesen werden. Zusammenfassend kann das Verfahren also beispielsweise wie folgt durchgeführt werden:

1. Start: Bei vorhandenen 2 Kalibrationspunkten bzw. Messpaaren (zum Beispiel kontinuierlicher Messstrom I, Blutglukose-Referenzwert $c_R$) wird eine Steigung sowie der Achsenabschnitt berechnet.

2. Bei Hinzunahme eines zusätzlichen Kalibrationspunkts in Form eines Messpaars gehe wie folgt vor:

a. Berechne die zusätzlichen Steigungen zwischen den vorhandenen Paaren.

b. Bilde aus mehreren, vorzugsweise allen, so berechneten Steigungen (bei 3 Paaren sind dies 2*3/2=3 Steigungen, bei n Punkten $n*(n-1)/2$) den Median. Dieser wird als neuer Wert für die wahrscheinliche Steigung der Kalibrationsgeraden verwendet.

c. Lege an jeden Kalibrationspunkt eine Gerade mit der unter 2b) berechneten wahrscheinlichen Steigung.

d. Berechne für mehrere Punkte, vorzugsweise für jeden Punkt, den Schnittpunkt mit der y-Achse (x-0).

e. Berechne aus allen so berechneten Achsenabschnitten den Median. Dieser ist ein neuer Schätzwert für den wahrscheinlichen Achsenabschnitt der Kalibrationsgeraden.

3. Die so berechnete Kalibrationsgerade dient zur Umrechnung von Messsignal in Blutglukose-Wert bis ein weiteres Kalibrationspaar zur Verfügung steht. Dann gehe zu 2.

Durch Vorgabe eines Referenzpunkts (zum Beispiel eines erwarteten Stromwerts bei einer Glukosekonzentration von 0 mg/dl) kann mit diesem Verfahren grundsätzlich auch eine Einpunktkalibration realisiert werden, wie in Figur 1A gezeigt.

Das Verfahren bietet durch die Verwendung robuster Medianschätzer den Vorteil, dass dieses gegen Ausreißer und Messfehler robust ist. Eine Bewertung der Güte eines aufgenommenen Kalibrationspunkts ist grundsätzlich nicht mehr notwendig, kann jedoch gegebenenfalls zusätzlich erfolgen.

Das oben anhand der Figuren 1A-1D beschriebene Verfahren kann durch verschiedene Ausgestaltungen weitergebildet werden. Eine Möglichkeit besteht in der Anwendung einer Datenkompression, welche jedoch grundsätzlich auch unabhängig eingesetzt werden kann. Eine derartige Datenkompression kann sowohl bei dem Kalibrierverfahren bei der Bestimmung der Kalibrationspunkte als auch bei der eigentlichen Messung, bei welcher auf eine Analytkonzentration in der Körperflüssigkeit geschlossen wird, oder bei einem dieser Verfahrensschritte, eingesetzt werden.

Eine erste Methode der Reduktion der Datenmenge besteht in der Bildung eines Medians aus mehreren einzelnen Messsignalen. So kann, um die Datenmenge zur Kalibration und/oder zur Auswertung zu reduzieren, eine Kompression von, beispielsweise im Subminutenbereich aufgenommenen, Messsignalen erfolgen, beispielsweise zu Werten im Minutenbereich. Dazu kann für jeweils einen Messzeitraum, eine Mehrzahl von Messsignalen aufgenommen werden,. Dabei ergibt n die Anzahl der verwendeten Werte im Subminutenbereich an. Aus diesen Werten, also den einzelnen Messsignalen des Messzeitraums, kann dann beispielsweise der Median berechnet werden und als komprimierter Wert für den jeweiligen Wert im Minutenbereich verwendet werden. Dieses komprimierte Messsignal kann dann beispielsweise im Kalibrierverfahren und/oder bei der eigentlichen Messung eingesetzt werden. Ebenso können in dem Zeitintervall des Messzeitraums Streumaße, wie beispielsweise die Standardabweichung und/oder das Quantilsmaß berechnet werden. Aus diesen Streumaßen kann beispielsweise der Signalzustand abgeleitet werden. Dies bezüglich kann beispielsweise auch auf US 7,389,133 B1 und auf US 2007/0016127 A1 verwiesen werden. Durch einen Vergleich zwischen den robusten Schätzern, beispielsweise dem Median und/oder den Quantilsmaßen, und parametrischen Schätzern, beispielsweise dem Mittelwert und/oder der Standardabweichung, können ebenfalls Aussagen über die Signalqualität getroffen werden.

Ein zweites, alternativ oder zusätzlich einsetzbares Verfahren zur Datenkompression, beispielsweise wiederum im Minutenbereich oder auf Basis eines anderen Messzeitraums, kann durch eine erneute Anwendung eines Regressionsverfahrens unter Verwendung eines oder mehrerer robuster Schätzverfahren durchgeführt werden. Dazu können beispielsweise, analog zur obigen Beschreibung der Figur 1C, aus der Mehrzahl von Messsignalen in dem zu komprimierenden Messzeitraum mögliche Steigungen zwischen allen oder mehreren der Messsignale des Messzeitraums, beispielsweise alle oder mehrere Steigungen zwischen jeweils zwei Werten im Subminutenbereich, berechnet werden und anschließend der Median aus allen Steigungen bestimmt werden. Geraden mit dieser medianen Steigung, die wiederum ein Beispiel einer wahrscheinlichen Steigung darstellt, können durch alle oder mehrere Strom-Zeit-Punkte des Messzeitraums gelegt werden und jeweils der Wert dieser Geraden zu einem bestimmten Zeitpunkt, welcher als repräsentativer Zeitpunkt für den Messzeitraum gewählt wird, zum Beispiel der Mitte des Zeitraums, berechnet bzw. bestimmt werden. Der Median aus allen so berechneten Strom-Zeit-Schätzern an dem repräsentativen Zeitpunkt, zu welchem die Auswertung durchgeführt wird, ist der Messwert für diesen Messzeitraum. Dieses Prinzip wird auch als Repeated Median-Prinzip bezeichnet. Die berechnete Geradensteigung, also die wahrscheinliche Steigung, kann zudem als Trendmaß für diesen Messzeitraum betrachtet werden.

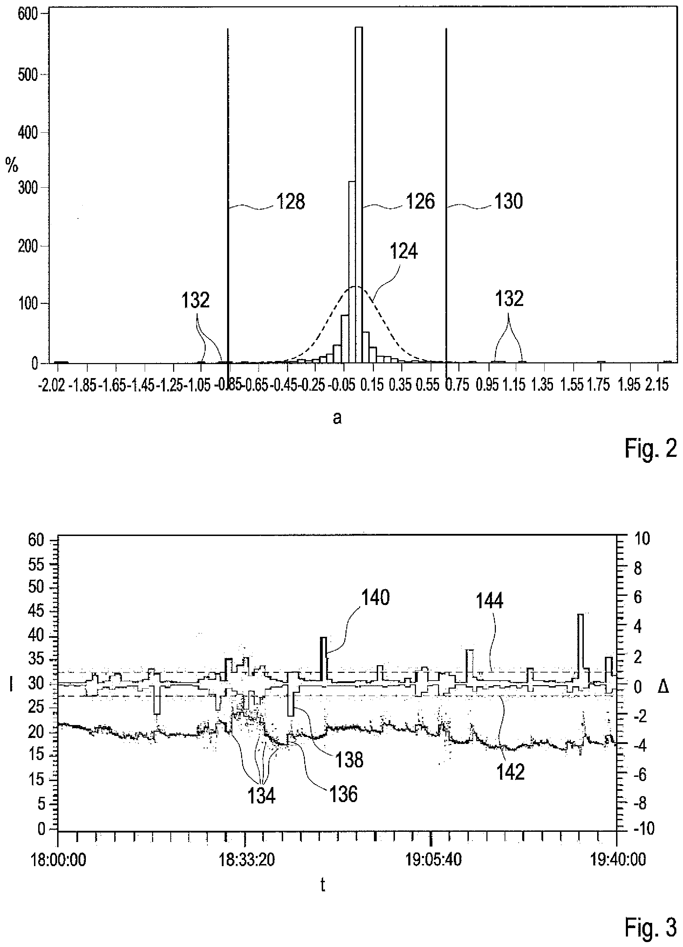

In einer weiteren möglichen Ausgestaltung des Verfahrens, welche anhand der Figur 2 erläutert werden soll, kann eine Beurteilung der Kalibrationsqualität erfolgen. So können beispielsweise zur Beurteilung der momentanen Qualität der Kalibration Konfidenzintervalle berechnet werden. Dies ermöglicht die Abschätzung, ob und wann eine weitere Kalibration erfolgen muss, also ob und wann das oben beschriebene Kalibrierverfahren erneut durchgeführt werden muss. Ferner wird eine Abschätzung ermöglicht, ob der Range (d.h. Bereich oder Toleranzbereich), also die Differenz zwischen dem maximalen Wert der Analytkonzentration und dem minimalen Wert der Analytkonzentration, die zur Durchführung des Kalibrierverfahrens herangezogen werden, für eine zuverlässige Kalibration ausreichend ist. Auch kann der Bereich der zur Berechnung zugelassenen Steigungen im Vorfeld eingeschränkt werden.

Ebenso können alte Kalibrationspunkte, deren Aufnahmezeitpunkt weiter als ein fest vorgegebenes Zeitintervall zurückliegt, von der Kalibration ausgeschlossen werden und so eine zeitliche Änderung des Systems kompensiert werden, da in diesem Fall eine neue Kalibrierung angeregt oder erzwungen wird.

So ist in Figur 2 eine mögliche statistische Verteilung der ermittelten Steigungen in einem Histogramm 126 aufgetragen. Die y-Achse bezeichnet dabei die Anzahl N der Zählungen einer bestimmten Steigung, wohingegen auf der x-Achse die Steigung aufgetragen ist. Wie aus Figur 2 ebenfalls erkennbar, kann eine untere Schwelle 128 und/oder eine obere Schwelle 130 vorgegeben werden, wobei Ausreißer (in Figur 2 mit der Bezugsziffer 132 bezeichnet, also Steigungen außerhalb des durch die Schwellen 128, 130 vorgegebenen Bereichs, verworfen werden, was entweder eine vollständige Ignorierung dieser Werte oder eine stark gering gewichtete Betrachtung dieser Werte beinhalten kann. Auf diese Weise kann eine Fehlkalibration vermieden werden, indem der Bereich der zulässigen Steigungen für die Bestimmung des Medians eingeschränkt wird.

Bevorzugt wird die Datenreduktion mit einer konstanten Reduktion ausgeführt. Weitere, alternative Ausführungsformen sehen vor, dass die Kalibrationsqualität zudem auch in eines oder beide der oben beschriebenen Verfahren zur Datenreduktion mit einfließen kann. So kann beispielsweise, wie oben dargestellt, eine Datenreduktion der Messsignale eines Messzeitraums auf ein oder wenige repräsentative Messwertpaare erfolgen, wobei jedes Messwertpaar beispielsweise einen Messwert und einen Zeitpunkt der Messung umfasst. Beispielsweise kann eine subminuten-basierte Menge an Messpunkten auf jeweils einen Wert im Minutenbereich komprimiert werden.
Dabei kann dieses repräsentative Messwertpaar allein abgespeichert werden, wobei beispielsweise das repräsentative Messwertpaar einen repräsentativen Messwert beziehungsweise ein repräsentatives Messsignal und einen repräsentativen Zeitpunkt des Messzeitraum umfassen kann. Zum Zwecke einer Informationserhaltung, zum Beispiel um eine automatische Fehlervermeidung (Failsafe) durchzuführen, können jedoch weitere Informationen zu diesem repräsentativen Messwertpaar hinzugenommen werden. Beispielsweise kann ein 5-dimensionaler Vektor verwendet werden, in welchem für jeden repräsentativen Zeitpunkt des Messzeitraums neben dem Median auch ein Mittelwert und/oder eine Standardabweichung und/oder ein 25%-Quantil und/oder 75%-) Quantil abgespeichert wird.

Alternativ oder zusätzlich zu der in Figur 2 beschriebenen Plausibilitätsbetrachtung, bei welcher automatisch Steigungen außerhalb des durch die Schwellen 128, 130 vorgegebenen Bereichs aussortiert bzw. verworfen werden können, können weitere Failsafe-Mechanismen vorgesehen sein. Diese können wahlweise bei dem Kalibrierverfahren und/oder bei der eigentlichen Messung zur Bestimmung der Analytkonzentration aus den Messsignalen eingesetzt werden.

In Figur 3 ist eine Messung von Roh-Messsignalen über einen Zeitraum hinweg dargestellt. Dabei bezeichnen die einzelnen Punkte die eigentlichen Messsignale. Die x-Achse stellt dabei die Zeit t dar. Auf der linken y-Achse sind die Messsignale I in willkürlichen Einheiten eingetragen. Die Messsignale sind in Figur 3 mit der Bezugsziffer 134 bezeichnet. Weiterhin ist in Figur 3, bezeichnet mit der Bezugsziffer 136, jeweils der Median der Messsignale 134 über 1 Minute aufgetragen, welcher sich als Stufenfunktion darstellt.

Weiterhin sind in Figur 3 zwei Differenzkurven dargestellt, die sich aus Differenzen verschiedener Quantile zum Median 136 ergeben. Diese Differenzen sind auf der rechten y-Achse in willkürlichen Einheiten angegeben. Dargestellt ist (Bezugsziffer 138) die Differenz des 0,25. Quantils zum Median 136 sowie (Bezugsziffer 140) die Differenz des 0,75. Quantils zum Median. Im Rahmen der vorliegenden Erfindung wird als Quantil der Ordnung p oder p. Quantil allgemein ein Wert bezeichnet, unterhalb dessen ein vorgegebener Anteil p aller Fälle der Verteilung liegt. Jeder Wert unterhalb von Qp unterschreitet diesen vorgegebenen Anteil. Dabei kann p eine beliebige reelle Zahl zwischen 0 und 1 sein.

Um eine automatische Fehlervermeidung zu erzielen, also um ein Failsafe-Verfahren zu realisieren, können diese Differenzen 138, 140 mit einem Schwellwertverfahren untersucht werden. Hierbei können ein oder mehrere Schwellwerte 142, 144 vorgegeben werden, und die Differenzen 138, 140 können mit diesen Schwellwerten 142, 144 verglichen werden. Dementsprechend können beispielsweise alle Differenzen 138, welche unterhalb des unteren Schwellwerts 142 liegen, verworfen werden, sowie alle Differenzen 140, welche oberhalb des oberen Schwellwerts 144 liegen. Auf diese Weise können beispielsweise Werte im Minutenbereich mit besonders hohen Abweichungen ausgeschlossen bzw. äußerst gering gewichtet werden. So lässt sich beispielsweise ein automatisches Failsafe-Verfahren realisieren. Allerdings ist hierbei vorzugsweise zu beachten, dass die Schwellwerte 142, 144 nicht derart gewählt werden, dass beispielsweise Werte im Minutenbereich während starker, durch die Änderung der Analytkonzentration verursachte Veränderungen, das heißt großer Steigungen, unberechtigterweise verworfen werden.

Ein weiterer Aspekt des erfindungsgemäßen Verfahrens besteht in der Option einer Anwendung einer Kalibrationsstrategie. Diese Kalibrationsstrategie kann grundsätzlich unabhängig von den übrigen Aspekten des vorgeschlagenen Verfahrens eingesetzt werden, ist jedoch besonders vorteilhaft in Kombination mit allen oder einigen der oben beschriebenen Ausgestaltungen des vorgeschlagenen Verfahrens. So kann eine Kalibrationsstrategie beispielsweise darin bestehen, den für den Anwender sichtbaren Analytkonzentrationsbereich, beispielsweise den Glukosebereich, in Abhängigkeit von der Qualität der Kalibration einzuschränken. So kann beispielsweise außerhalb dieses Bereichs eine Anzeige auf "Untergrenze unterschritten" oder "Obergrenze überschritten" umgeschaltet werden. Alternativ oder zusätzlich kann die Kalibrationsstrategie beispielsweise auch darin bestehen, den Anwender zu einer erneuten Kalibration aufzufordern. Wiederum alternativ oder zusätzlich kann die Kalibrationsstrategie auch darin bestehen, dass dem Anwender signalisiert wird, wenn ein günstiger Kalibrationszeitpunkt, also ein günstiger Zeitpunkt zum Durchführen des oben beschriebenen Kalibrierverfahrens und/oder eines anderen Kalibrierverfahrens, vorliegt. Beispielsweise kann ein günstiger Kalibrationszeitpunkt dann vorliegen, wenn momentan eine geringe Dynamik in den Messsignalen vorliegt und/oder wenn ein zu erwartender Analytkonzentrationswert stark von den bisherigen Werten abweicht. Auch andere Arten von günstigen Kalibrationszeitpunkten sind möglich.

In einem weiteren Aspekt der vorliegenden Erfindung kann eine Glättung von Rohwerten vorgenommen werden, beispielsweise mittels mindestens eines Exponentialfilters. Diese Glättung kann beispielsweise zur Durchführung des Kalibrierverfahrens verwendet werden und/oder für die eigentliche Messung, um aus den Messsignalen geglättete Messsignale zu gewinnen. Beispielsweise kann ein Exponentialfilter eingesetzt werden, wie er beispielsweise in Hartung:

Statistik. Lehr- und Handbuch der Angewandten Statistik, 14. Auflage, Kapitel XII, 1.3.4.: Exponentielles Glätten, S. 672-673, beschrieben wird.

Um die Rohwerte der Messsignale zu glätten, kann beispielsweise ein Verfahren eingesetzt werden, bei welchem $x_1,..., x_m$ die zu glättenden Werte darstellen. Dann ergeben sich die geglätteten Werte $\tilde{x}_1;...;\tilde{x}_m$ beispielsweise durch folgende Vorschrift:

$$\tilde{x}_1 = \begin{cases} x_1, fallsStatus(x_1) = g\ddot{u}ltig, \\ 0 sonst. \end{cases}$$

und

$$\tilde{x}_{n+1} = \begin{cases} \alpha x_{n+1} + (1-\alpha)\tilde{x}_n, fallsStatus(x_{n+1}) = g\ddot{u}ltig, \\ \tilde{x}_n sonst. \end{cases} ; n = 1,...,m-1.$$

Der Status eines Werts kann beispielsweise durch das oben beschriebene Plausibilitätsverfahren erhalten werden. So kann beispielsweise der Status auf ungültig gesetzt werden, wenn in dem in Figur 3 gezeigten Plausibilitätsverfahren einer der Schwellwerte 142, 144 unterschritten bzw. überschritten wird. Ansonsten wird ein Status auf gültig gesetzt. Der Wert $\alpha$ bezeichnet hingegen einen Glättungsfaktor, welcher im Intervall zwischen 0 und 1 liegt. Für $\alpha = 0$ ergibt sich beispielsweise konstant der Anfangswert, wohingegen sich für $\alpha = 1$ keine Glättung ergibt. Der Glättungsfaktor $\alpha$ kann beispielsweise in einem Datenspeicher des Messgeräts 110 festgelegt werden, beispielsweise einem persistenten Datenspeicher. Wie oben bereits ausgeführt, kann das Messgerät 110 dabei auch dezentral ausgestaltet sein, so dass auch ein Messsystem mit mehreren einzelnen Komponenten, beispielsweise miteinander in einer unidirektionalen oder bidirektionalen Kommunikationsverbindung stehenden Komponenten, wie beispielsweise kontinuierliche oder diskret messende Sensoren, Steuergeräten, externen Speicher oder ähnliche Komponenten, vom Begriff des Messgeräts 110 umfasst sein kann.

Weiterhin kann, sowohl bei dem oben beschriebenen Kalibrierverfahren als auch bei der eigentlichen Messung zur Ermittlung der Analytkonzentration oder lediglich bei einem der genannten Verfahrensschritte, auch mindestens ein Linearisierungsschritt durchgeführt werden. Sollte sich beispielsweise ein Strom-Analyt-Konzentrationszusammenhang (oder ein entsprechendes Verhältnis bzw. Funktion) als (zumindest abschnittsweise) nicht linear herausstellen, so kann in einem Zwischenschritt ein derartiger Linearisierungsschritt durchgeführt werden. Dabei können beispielsweise die rohen Messsignale, beispielsweise die rohen Stromwerte, über eine Linearisierungsfunktion so abgebildet werden, dass die umgerechneten Messsignale einen linearen Zusammenhang mit der Analytkonzentration in der Körperflüssigkeit aufweisen. Dieses Verfahren bietet sich insbesondere für eine Vorkalibration an. Die entsprechende Umrechnungsfunktion kann beispielsweise an einem Messstand chargenabhängig bestimmt und in einem Datenspeicher der Steuerung 114, beispielsweise wiederum einem persistenten Datenspeicher, abgelegt werden. Die eigentliche Feinkalibration kann dann In Vivo, wie oben beschrieben, durchgeführt werden.

In Figur 4 ist schließlich ein mögliches schematisches Ablaufdiagramm eines Ausführungsbeispiels eines erfindungsgemäßen Verfahrens zum Betrieb eines Messgeräts 110 zur Erfassung mindestens eines Analyten in einer Körperflüssigkeit dargestellt. Dabei bezeichnet die Bezugsziffer 146 die Generierung eines oder mehrerer Messsignale, insbesondere als Funktion der Zeit. Beispielsweise können im Sekundenabstand Strom-Messsignale des Messgeräts 110, insbesondere des Blutglukose-Sensors 112, aufgenommen werden. Auf diese Messsignale kann, was mit der Bezugsziffer 148 bezeichnet ist, optional eine Glättung angewandt werden, was eine Filterung beinhalten kann. Beispielsweise kann hier ein Exponentialfilter eingesetzt werden, beispielsweise gemäß der oben beschriebenen Funktion.

In Verfahrensschritt 150 kann optional eine Datenkompression erfolgen, beispielsweise gemäß einem oder mehrerer der oben beschriebenen Verfahren. Auf diese Weise kann beispielsweise ein "Downsizing" von im Subminutenbereich aufgenommenen Messwerten auf Werte im Minutenbereich.

Anschließend kann optional in Verfahrensschritt 152 eine Plausibilitätsbetrachtung durchgeführt werden, insbesondere im Rahmen eines so genannten Failsafe-Verfahrens. Hierbei können, wie oben beispielsweise anhand der Figur 3 beschrieben, unplausible Messsignale verworfen werden.

Anschließend kann in Verfahrensschritt 154 optional eine Übermittlung der derart bearbeiteten Messsignale erfolgen. Beispielsweise kann eine Übermittlung an die Steuerung 114 und/oder einen Datenspeicher der Steuerung 114 oder eine weiterverarbeitende Recheneinheit erfolgen.

Anschließend kann in Verfahrensschritt 156 erneut optional eine Glättung durchgeführt werden, beispielsweise wiederum eine Filterung.

In Verfahrensschritt 158 werden Kalibrationspunkte gebildet, in dem den Messsignalen bzw. den aufbereiteten Messsignalen (beispielsweise optional geglätteten und/oder komprimierten und/oder auf andere Weise ausgewerteten Messsignalen) entsprechende Referenzwerte zugehöriger Referenzmessungen zugeordnet werden. Dieser Verfahrensschritt 158 kann auch als Synchronisation bezeichnet werden.

Anschließend kann in Verfahrensschritt 160 das Kalibrierverfahren zur prospektiven Kalibrierung der Kalibrationspunkte durchgeführt werden. Bei diesem Kalibrierverfahren wird, beispielsweise wie oben beschrieben, ein Zusammenhang zwischen den Messsignalen, gegebenenfalls also den aufbereiteten Messsignalen, und der Analytkonzentration in der Körperflüssigkeit hergestellt. Beispielsweise kann dies, wie oben beschrieben, unter Ermittlung der mindestens einen wahrscheinlichen Steigung und optional des mindestens einen wahrscheinlichen Achsenabschnitts erfolgen. Dieser ermittelte Zusammenhang kann beispielsweise in dem Messgerät 110 anschließend für eine Messung eingesetzt werden, um aus Messsignalen die Analytkonzentration zu bestimmen. Diese kann beispielsweise auf einer Anzeige 162 des Messgeräts 110 dargestellt werden.

Wie oben dargestellt, kann das Verfahren optional eine Kalibrationsstrategie umfassen. Diese Kalibrationsstrategie kann beispielsweise eine Empfehlung zur Durchführung eines Kalibrierverfahrens umfassen. Dies ist in Figur 4 mit der Bezugsziffer 164 bezeichnet. Diese Empfehlung kann beispielsweise wiederum auf einer Anzeige 162 des Messgeräts 110 dargestellt werden. Insbesondere kann eine Kalibrationsstrategie verwendet werden, wie sie oben anhand der Zustände (A) - (E) beschrieben ist.

Während bei der prospektiven Kalibrierung des Messgeräts 110 ein Zusammenhang zwischen den Messsignalen und der Analytkonzentration für zukünftige Messungen ermittelt wird, kann optional auch eine retrospektive Kalibrierung durchgeführt werden. Dies ist in Figur 4 mit der Bezugsziffer 166 bezeichnet. Bei der retrospektiven Kalibrierung können beispielsweise zwischenzeitlich hinzu gekommene Kalibrierungsinformationen, welche beispielsweise zu einer genaueren und unzuverlässigeren Kalibrierung geführt haben, genutzt werden, um ältere Messsignale erneut auszuwerten. Dies kann beispielsweise im Messgerät selbst erfolgen und/oder, wie in Figur 4 dargestellt, auf einem weiteren Computer, beispielsweise einem Arztcomputer 168. Auf diese Weise können nachträglich Messungen mit höherer Genauigkeit ausgewertet werden.

**Bezugszeichenliste**

| | |
|---|---|
| 110 | Messgerät zur Erfassung eines Analyten in einer Körperflüssigkeit |
| 112 | kontinuierlich messender Blutglukose-Sensor |
| 114 | Steuerung |
| 116 | Computer |
| 118 | Verbindung |
| 120 | Empfang von Referenzwerten |
| 122 | Referenzmessgerät |
| 126 | Histogramm der Steigungen |
| 128 | untere Schwelle |
| 130 | obere Schwelle |
| 132 | Ausreißer |
| 134 | Messsignale |
| 136 | Median der Messsignale |
| 138 | Differenz 25. Quantil zum Median |
| 140 | Differenz 75. Quantil zum Median |
| 142 | unterer Schwellwert |
| 144 | oberer Schwellwert |
| 146 | Generierung Messsignal |
| 148 | Glättung |
| 150 | Datenkompression |
| 152 | Plausibilitätsbetrachtung |
| 154 | Übermittlung |
| 156 | Glättung |
| 158 | Bildung Kalibrationspunkte |
| 160 | Kalibrierverfahren |
| 162 | Anzeige |
| 164 | Empfehlung zur Durchführung Kalibrierverfahren |

**EP 2 470 913 B1**

166    Retrospektive Kalibrierung
168    Arztcomputer

**Patentansprüche**

1. Verfahren zum Betrieb eines Messgeräts (110) zur Erfassung mindestens eines Analyten in einer Körperflüssigkeit, insbesondere mittels mindestens eines kontinuierlich messenden Blutglukose-Sensors (112),

   - wobei mindestens ein Kalibrierverfahren zur prospektiven Kalibrierung des Messgeräts (110) durchgeführt wird, wobei in dem Kalibrierverfahren mindestens drei Kalibrationspunkte erfasst werden, wobei jeder Kalibrationspunkt mindestens ein Messsignal des Messgeräts (110) und mindestens einen Referenzwert einer zugehörigen Referenzmessung umfasst, wobei der mindestens eine Referenzwert eine Information über eine tatsächlich vorhandene Konzentration des Analyten in der Körperflüssigkeit liefert, wobei eine Mehrzahl möglicher Steigungen zwischen den Kalibrationspunkten ermittelt wird, wobei mittels mindestens eines robusten Schätzverfahrens unter Verwendung einer Bildung mindestens eines Medians aus der Mehrzahl möglicher Steigungen mindestens eine wahrscheinliche Steigung bestimmt wird, und das robuste Schätzverfahren ein statistisches Schätzverfahren ist, welches auch bei dem Auftreten von Ausreißern oder nur approximativer Gültigkeit von Verteilungsannahmen noch stabile statistische Schätzer liefert, und ferner das Schätzverfahren insbesondere ein Schätzverfahren ist, das auf einem oder mehreren Permutationsalgorithmen und/oder auf einem oder mehreren Sortieralgorithmen basiert;
   - wobei weiterhin mindestens eine Messung durchgeführt wird, wobei bei der Messung unter Verwendung der wahrscheinlichen Steigung aus mindestens einem Messsignal des Messgeräts (110) und der wahrscheinlichen Steigung auf eine Konzentration des Analyten in der Körperflüssigkeit geschlossen wird.

2. Verfahren nach dem vorhergehenden Anspruch, wobei bei dem Kalibrierverfahren weiterhin eine Mehrzahl von Geraden mit der wahrscheinlichen Steigung durch eine Mehrzahl der Kalibrationspunkte, vorzugsweise durch alle Kalibrationspunkte, bestimmt wird, wobei die Achsenabschnitte der Geraden bestimmt werden, wobei mittels mindestens eines robusten Schätzverfahrens, insbesondere unter Verwendung einer Bildung mindestens eines Medians, aus den Achsenabschnitten mindestens ein wahrscheinlicher Achsenabschnitt bestimmt wird, wobei bei der Messung der wahrscheinliche Achsenabschnitt verwendet wird.

3. Verfahren nach einem der vorhergehenden Ansprüche, wobei das Kalibrierverfahren wiederholt durchgeführt wird, insbesondere zu verschiedenen Zeiten, wobei nach einer Wiederholung des Kalibrierverfahrens eine neue wahrscheinliche Steigung und vorzugsweise ein neuer wahrscheinlicher Achsenabschnitt bestimmt wird und die neue wahrscheinliche Steigung und vorzugsweise der neue wahrscheinliche Achsenabschnitt in mindestens einer nachfolgenden Messung verwendet werden.

4. Verfahren nach einem der vorhergehenden Ansprüche, wobei bei dem Kalibrierverfahren eine Plausibilitätsbetrachtung durchgeführt wird, wobei bei der Plausibilitätsbetrachtung unrealistische Kalibrationspunkte und/oder unrealistische Steigungen und/oder unrealistische Achsenabschnitte verworfen werden.

5. Verfahren nach einem der vorhergehenden Ansprüche, wobei bei dem robusten Schätzverfahren eine Bildung mindestens eines Medians verwendet wird, wobei zur Bildung des Medians ein Sortieralgorithmus und/oder ein Permutationsalgorithmus verwendet werden.

6. Verfahren nach einem der vorhergehenden Ansprüche, wobei mindestens eine aktuelle Kalibrationsqualität bestimmt wird, wobei die Kalibrationsqualität insbesondere mindestens ein Konfidenzintervall umfasst.

7. Verfahren nach einem der vorhergehenden Ansprüche, wobei ein Benutzer zur Durchführung des Kalibrierverfahrens aufgefordert wird, vorzugsweise automatisch.

8. Verfahren nach dem vorhergehenden Anspruch, wobei der Benutzer zur Durchführung des Kalibrierverfahrens aufgefordert wird, wenn mindestens ein aktuelles Messsignal in einem Bereich liegt, in dem das Kalibrierverfahren noch nicht mit ausreichender Kalibrationsqualität durchgeführt wurde.

9. Verfahren nach einem der vorhergehenden Ansprüche, wobei ein Benutzer darüber informiert wird, wenn ein aktuelles Messsignal in einem Bereich liegt, in dem keine ausreichende Kalibrationsqualität vorliegt.

10. Verfahren nach einem der vorhergehenden Ansprüche, wobei in regelmäßigen oder unregelmäßigen Abständen Kalibrationspunkte, welche älter sind als eine vorgegebene Zeitschwelle, verworfen werden.

11. Verfahren nach einem der vorhergehenden Ansprüche, wobei die Kalibrationspunkte und/oder die Messsignale mindestens einem Glättungsverfahren unterzogen werden, insbesondere einem Glättungsverfahren mittels eines Exponentialfilters.

12. Verfahren nach einem der vorhergehenden Ansprüche, wobei die Kalibrationspunkte und/oder die Messsignale mindestens einem Linearisierungsschritt unterworfen werden, wobei durch den Linearisierungsschritt ein zumindest näherungsweise linearer Zusammenhang zwischen den Messsignalen und den Referenzwerten hergestellt wird.

13. Verfahren zum Betrieb eines Messgeräts (110) zur Erfassung mindestens eines Analyten in einer Körperflüssigkeit nach einem der vorhergehenden Verfahrensansprüche, wobei bei einer Messung eine Mehrzahl von Messsignalen des Messgeräts (110) über einen Messzeitraum erfasst wird, wobei weiterhin eine Datenreduktion durchgeführt wird, wobei bei der Datenreduktion ein Datenreduktionsverfahren angewandt wird, demgemäß

    - die Messsignale mit den zugehörigen Messzeitpunkten zu Messwertpaaren zusammengefasst werden, wobei eine Mehrzahl möglicher Steigungen zwischen den Messwertpaaren ermittelt wird, wobei mittels mindestens eines robusten Schätzverfahrens, insbesondere unter Verwendung mindestens eines Medians, aus der Mehrzahl möglicher Steigungen eine wahrscheinliche Steigung ermittelt wird, wobei eine Mehrzahl von Geraden mit der wahrscheinlichen Steigung durch die Messwertpaare gebildet wird, wobei mindestens ein repräsentativer Zeitpunkt für den Messzeitraum gewählt wird, wobei die Funktionswerte der Geraden für den repräsentativen Zeitpunkt ermittelt werden, wobei aus den Funktionswerten mittels mindestens eines robusten Schätzverfahrens mindestens ein wahrscheinlicher Funktionswert ermittelt wird, wobei die Mehrzahl der Messwertpaare durch mindestens ein repräsentatives Messwertpaar mit dem mindestens einen wahrscheinlichen Funktionswert und dem mindestens einen repräsentativen Zeitpunkt ersetzt wird

und insbesondere ferner das folgende Datenreduktionsverfahren angewandt wird:

    - mittels mindestens eines robusten Schätzverfahrens, insbesondere unter Verwendung einer Bildung mindestens eines Medians, wird aus der Mehrzahl von Messsignalen mindestens ein wahrscheinliches Messsignal für den Messzeitraum bestimmt, wobei die Mehrzahl von Messsignalen durch das wahrscheinliche Messsignal ersetzt wird.

14. Verfahren nach dem vorhergehenden Anspruch, wobei weiterhin bei der Datenreduktion aus der Mehrzahl von Messsignalen mindestens ein Streumaß bestimmt wird, insbesondere eine Standardabweichung und/oder ein Quantilsmaß.

15. Verfahren nach einem der beiden vorhergehenden Ansprüche, wobei weiterhin mindestens eine Aussage über eine Signalqualität der Mehrzahl von Messsignalen getroffen wird, insbesondere durch einen Vergleich zwischen robusten Schätzern der Messsignale und parametrischen Schätzern der Messsignale.

16. Verfahren nach einem der drei vorhergehenden Ansprüche, wobei weiterhin aus der Mehrzahl von Messsignalen ein Trend bestimmt wird.

17. Verfahren zum Betrieb eines Messgeräts (110) zur Erfassung mindestens eines Analyten in einer Körperflüssigkeit nach einem der vorhergehenden Verfahrensansprüche, wobei in dem mindestens einem Kalibrierverfahren eine Kalibrierung von Messsignalen des Messgeräts (110) gegen Referenzwerte zugehöriger Referenzmessungen durchgeführt wird, wobei eine Kalibrationsstrategie angewandt wird, wobei die Kalibrationsstrategie eine oder mehrere der folgenden Verfahrensschritte umfasst:

    - in Abhängigkeit von einer aktuellen Kalibrationsqualität wird ein für eine Messung verwendbarer Bereich von Messsignalen des Messgeräts (110) eingeschränkt, wobei insbesondere bei Erfassung von Messsignalen außerhalb des durch Kalibrationswerte abgedeckten oder verwendbaren Bereiches oder eines Toleranzbereichs, der auf den Kalibrationswerten basiert, ein Hinweis an einen Benutzer erfolgt;
    - ein Benutzer wird zur erneuten Durchführung des Kalibrierverfahrens aufgefordert;
    - ein Benutzer wird darüber informiert, dass ein günstiger Zeitpunkt zur Durchführung des Kalibrierverfahrens vorliegt, insbesondere bei aktuell geringer Signaldynamik der Messsignale und/oder bei einer zu erwartenden starken Abweichung der Konzentration des mindestens einen Analyten in der Körperflüssigkeit von bisherigen Analytkonzentration;
- ein Benutzer wird in regelmäßigen oder unregelmäßigen Abständen oder zu vorgegebenen Zeitpunkten zur erneuten Durchführung des Kalibrationsverfahrens aufgefordert.

**18.** Computerprogramm mit Programmcode zur Durchführung des Verfahrens nach einem der vorhergehenden Verfahrensansprüche, wenn das Programm in einem Computer ausgeführt wird, insbesondere in einem Computer einer Steuerung eines Messgeräts (110) zur Erfassung mindestens eines Analyten in einer Körperflüssigkeit.

**19.** Messgerät (110) zur Erfassung mindestens eines Analyten in einer Körperflüssigkeit, insbesondere umfassend mindestens einen kontinuierlich messenden Blutglukose-Sensor (112), wobei das Messgerät (110) eingerichtet ist, um mindestens ein Messsignal entsprechend einer Konzentration des Analyten in der Körperflüssigkeit zu erzeugen, wobei das Messgerät (110) weiterhin eingerichtet ist, um mittels einer unabhängigen Referenzmessung erfasste Referenzwerte zu empfangen, wobei das Messgerät (110) mindestens eine Steuerung (114) aufweist, insbesondere eine Steuerung (114) mit mindestens einem Computer, wobei die Steuerung (114) eingerichtet ist, um ein Verfahren nach einem der vorhergehenden Verfahrensansprüche durchzuführen.

**Claims**

**1.** Method for operating a measuring device (110) for detecting at least one analyte in a bodily fluid, in particular using at least one continuously measuring blood glucose sensor (112),

- wherein at least one calibration method is performed for prospective calibration of the measuring device (110), wherein at least three calibration points are detected in the calibration method, wherein each calibration point comprises at least one measurement signal of the measuring device (110) and at least one reference value of an associated reference measurement, wherein the at least one reference value supplies information about a concentration of the analyte actually present in the bodily fluid, wherein a plurality of possible slopes are determined between the calibration points, wherein at least one probable slope is determined using at least one robust estimation method using a formation of at least one median from the plurality of possible slopes, and the robust estimation method is a statistical estimation method, which still supplies stable statistical estimators even when outliers occur or when distribution assumptions are only approximately valid, and the estimation method further is an estimation method, in particular, that is based on one or more permutation algorithms and/or on one or more sort algorithms;
- wherein, furthermore, at least one measurement is performed, wherein a concentration of the analyte in the bodily fluid is determined during the measurement using the probable slope of at least one measurement signal of the measuring device (110) and the probable slope.

**2.** Method according to the preceding claim, wherein, furthermore, a plurality of straight lines with the probable slope through a plurality of calibration points, preferably through all calibration points, are determined during the calibration method, wherein the intercepts of the straight lines are determined, wherein at least one probable intercept is determined from the intercepts by means of at least one robust estimation method, more particularly using a formation of at least one median, wherein the probable intercept is used during the measurement.

**3.** Method according to any one of the preceding claims, wherein the calibration method is performed repeatedly, in particular at different times, wherein a new probable slope and, preferably, a new probable intercept are determined following a repetition of the calibration method and the new probable slope and, preferably, the new probable intercept are used in at least one subsequent measurement.

**4.** Method according to any one of the preceding claims, wherein a plausibility check is performed during the calibration method, wherein unrealistic calibration points and/or unrealistic slopes and/or unrealistic intercepts are discarded during the plausibility check.

**5.** Method according to any one of the preceding claims, wherein a formation of at least one median is used in the robust estimation method, wherein a sort algorithm and/or permutation algorithm are used to form the median.

**6.** Method according to any one of the preceding claims, wherein at least one current calibration quality is determined, wherein the calibration quality comprises at least one confidence interval, in particular.

7. Method according to any one of the preceding claims, wherein a user is prompted, preferably automatically, to perform the calibration method.

8. Method according to the preceding claim, wherein the user is prompted to perform the calibration method if at least one current measurement signal lies in a range in which the calibration method has not yet been performed with sufficient calibration quality.

9. Method according to any one of the preceding claims, wherein a user is informed if a current measurement signal lies in a range in which no sufficient calibration quality is present.

10. Method according to any one of the preceding claims, wherein calibration points that are older than a predetermined time threshold are discarded at regular or irregular intervals.

11. Method according to any one of the preceding claims, wherein the calibration points and/or the measurement signals are subjected to at least one smoothing method, more particularly a smoothing method by means of an exponential filter.

12. Method according to any one of the preceding claims, wherein the calibration points and/or the measurement signals are subjected to at least one linearization step, wherein an at least approximately linear relationship is established between the measurement signals and the reference values by way of the linearization step.

13. Method for operating a measuring device (110) for detecting at least one analyte in a bodily fluid according to any one of the preceding method claims, wherein a plurality of measurement signals of the measuring device (110) are detected over a measurement time period during a measurement, wherein, furthermore, a data reduction is performed, wherein a data reduction method is applied during the data reduction, according to which

    - the measurement signals are combined with the associated measurement times to form measurement value pairs, wherein a plurality of possible slopes are determined between the measurement value pairs, wherein a probable slope is determined from the plurality of possible slopes by means of at least one robust estimation method, in particular using at least one median, wherein a plurality of straight lines with the probable slope are formed through the measurement value pairs, wherein at least one representative time is chosen for the measurement time period, wherein the functional values of the straight lines are ascertained for the representative time, wherein at least one probable functional value is determined from the functional values using a robust estimation method, wherein the plurality of the measurement value pairs are replaced by at least one representative measurement value pair with the at least one probable functional value and the at least one representative time,

    and, in particular, the following data reduction method is further applied:

    - a probable measurement signal for the measurement time period is determined using at least one robust estimation method, in particular using a formation of at least one median, from the plurality of measurement signals, wherein the plurality of measurement signals are replaced by the probable measurement signal.

14. Method according to the preceding claim, wherein, furthermore, at least one measure of dispersion, more particularly a standard deviation and/or a quantile measure, is determined from the plurality of measurement signals during the data reduction.

15. Method according to any one of the two preceding claims, wherein, furthermore, at least one statement is made about a signal quality of the plurality of measurement signals, in particular by a comparison between robust estimators of the measurement signals and parametric estimators of the measurement signals.

16. Method according to any one of the three preceding claims, wherein, furthermore, a trend is determined from the plurality of measurement signals.

17. Method for operating a measuring device (110) for detecting at least one analyte in a bodily fluid according to any one of the preceding method claims, wherein a calibration of measurement signals of the measuring device (110) is performed in relation to reference values of associated reference measurements in the at least one calibration method, wherein a calibration strategy is applied, wherein the calibration strategy comprises one or more of the following method steps:

- a range of measurement signals of the measuring device (110) that is applicable for a measurement is restricted depending on a current calibration quality, wherein a notification is provided to a user, in particular when detecting measurement signals outside of the range that is covered by calibration values or outside of the applicable range, or outside of a tolerance range that is based on the calibration values;
- a user is requested to perform the calibration method again;
- a user is informed that an expedient time is present for performing the calibration method, particularly in the case of currently low signal dynamics of the measurement signals and/or in the case of an expected strong deviation of the concentration of the at least one analyte in the bodily fluid from previous analyte concentration;
- a user is requested to perform the calibration method again at regular or irregular intervals or predetermined times.

18. Computer program with program code for performing the method according to any one of the preceding method claims when the program is executed on a computer, in particular on a computer of a controller of a measuring device (110) for detecting at least one analyte in a bodily fluid.

19. Measuring device (110) for detecting at least one analyte in a bodily fluid, in particular comprising at least one continuously measuring blood glucose sensor (112), wherein the measuring device (110) is configured to produce at least one measurement signal in accordance with a concentration of the analyte in the bodily fluid, wherein the measuring device (110) is further designated to receive reference values detected by means of an independent reference measurement, wherein the measuring device (110) has at least one controller (114), in particular a controller (114) with at least one computer, wherein the controller (114) is configured to perform a method according to any one of the preceding method claims.

**Revendications**

1. Procédé de fonctionnement d'un appareil de mesure (110) pour la détection d'au moins un analyte dans un liquide corporel, en particulier au moyen d'un détecteur de glucose sanguin à mesure continue (112),

- dans lequel on exécute au moins un procédé de calibrage destiné au calibrage prospectif de l'appareil de mesure (110), dans lequel on détecte dans le procédé de calibrage au moins trois points de calibrage, dans lequel chaque point de calibrage comprend au moins un signal de mesure de l'appareil de mesure (110) et au moins une valeur de référence d'une mesure de référence correspondante, dans lequel ladite au moins une valeur de référence fournit une information sur la concentration effectivement présente de l'analyte dans le liquide corporel, dans lequel on détermine une multiplicité de pentes possibles entre les points de calibrage, dans lequel on détermine au moyen d'au moins un solide procédé d'estimation en utilisant une formation d'au moins une médiane au moins une pente probable parmi la multiplicité de pentes possibles, et le solide procédé d'estimation est un procédé d'estimation statistique, qui fournit encore, même en cas de survenance de données aberrantes ou de validité seulement approximative d'hypothèses de distribution, des estimateurs statistiques stables, et en outre le procédé d'estimation est en particulier un procédé d'estimation qui se base sur un ou plusieurs algorithme(s) de permutation et/ou sur un ou plusieurs algorithme(s) de tri;
- dans lequel on effectue en outre au moins une mesure, dans lequel lors de la mesure avec utilisation de la pente probable on déduit une concentration de l'analyte dans le liquide corporel à partir d'au moins un signal de mesure de l'appareil de mesure (110) et de la pente probable.

2. Procédé selon la revendication précédente, dans lequel on détermine en outre lors du procédé de calibrage une multiplicité de droites avec la pente probable par une multiplicité des points de calibrage, de préférence par tous les points de calibrage, dans lequel on détermine les parties axiales des droites, dans lequel on détermine au moyen d'au moins un solide procédé d'estimation, en particulier en utilisant une formation d'au moins une médiane, au moins une partie axiale probable parmi les parties axiales, dans lequel on utilise lors de la mesure la partie axiale probable.

3. Procédé selon l'une quelconque des revendications précédentes, dans lequel on exécute le procédé de calibrage de façon répétée, en particulier à des instants différents, dans lequel on détermine après une répétition du procédé de calibrage une nouvelle pente probable et de préférence une nouvelle partie axiale probable et on utilise la nouvelle pente probable et de préférence la nouvelle partie axiale probable dans au moins une mesure suivante.

4. Procédé selon l'une quelconque des revendications précédentes, dans lequel lors du procédé de calibrage on effectue une vérification de plausibilité, dans lequel lors de la vérification de plausibilité on rejette des points de calibrage irréalistes et/ou des pentes irréalistes et/ou des parties axiales irréalistes.

5. Procédé selon l'une quelconque des revendications précédentes, dans lequel lors du solide procédé d'estimation on utilise une formation d'au moins une médiane, dans lequel on utilise pour la formation de la médiane un algorithme de tri et/ou un algorithme de permutation.

6. Procédé selon l'une quelconque des revendications précédentes, dans lequel on détermine une qualité de calibrage actuelle, dans lequel la qualité de calibrage comprend en particulier au moins un intervalle de confiance.

7. Procédé selon l'une quelconque des revendications précédentes, dans lequel on invite un utilisateur, de préférence automatiquement, à exécuter le procédé de calibrage.

8. Procédé selon la revendication précédente, dans lequel on invite l'utilisateur à exécuter le procédé de calibrage, lorsqu'au moins un signal de mesure actuel se situe dans une plage, dans laquelle le procédé de calibrage n'a pas encore été exécuté avec une qualité de calibrage satisfaisante.

9. Procédé selon l'une quelconque des revendications précédentes, dans lequel on informe un utilisateur, quand un signal de mesure actuel se situe dans une plage, dans laquelle une qualité de calibrage satisfaisante n'est pas présente.

10. Procédé selon l'une quelconque des revendications précédentes, dans lequel on rejette à intervalles réguliers ou irréguliers des points de calibrage, qui sont plus anciens qu'un seuil de temps prédéterminé.

11. Procédé selon l'une quelconque des revendications précédentes, dans lequel on soumet les points de calibrage et/ou les signaux de mesure à au moins un procédé de lissage, en particulier à un procédé de lissage au moyen d'un filtre exponentiel.

12. Procédé selon l'une quelconque des revendications précédentes, dans lequel on soumet les points de calibrage et/ou les signaux de mesure à au moins une étape de linéarisation, dans lequel on établit par l'étape de linéarisation une relation au moins approximativement linéaire entre les signaux de mesure et les valeurs de référence.

13. Procédé de fonctionnement d'un appareil de mesure (110) pour la détection d'au moins un analyte dans un liquide corporel selon l'une quelconque des revendications de procédé précédentes, dans lequel on détecte lors d'une mesure une multiplicité de signaux de mesure de l'appareil de mesure (110) sur une période de mesure, dans lequel on opère en outre une réduction de données, dans lequel on applique lors de la réduction de données un procédé de réduction de données, selon lequel

- on rassemble les signaux de mesure avec les instants de mesure correspondants en paires de valeurs de mesure, dans lequel on détermine une multiplicité de pentes possibles entre les paires de valeurs de mesure, dans lequel on détermine au moyen d'au moins un solide procédé d'estimation, en particulier avec utilisation d'au moins une médiane, une pente probable parmi la multiplicité de pentes possibles, dans lequel on forme une multiplicité de droites avec la pente probable par les paires de valeurs de mesure, dans lequel on choisit au moins un instant représentatif pour la période de mesure, dans lequel on détermine les valeurs de fonction des droites pour l'instant représentatif, dans lequel on détermine parmi les valeurs de fonction au moins une valeur de fonction probable au moyen d'au moins un solide procédé d'estimation, dans lequel on remplace la multiplicité des paires de valeurs de mesure par au moins une paire de valeurs de mesure représentative avec ladite au moins une valeur de fonction probable et ledit au moins un instant représentatif

et en particulier on applique en outre le procédé de réduction de données suivant:

- on détermine parmi la multiplicité de signaux de mesure, au moyen d'au moins un solide procédé d'estimation, en particulier avec utilisation d'une formation d'au moins une médiane, au moins un signal de mesure probable pour la période de mesure, dans lequel on remplace la multiplicité de signaux de mesure par le signal de mesure probable.

14. Procédé selon la revendication précédente, dans lequel on détermine en outre, lors de la réduction de données, parmi la multiplicité de signaux de mesure, au moins une mesure de dispersion, en particulier un écart-type et/ou une mesure de quantile.

**15.** Procédé selon l'une des deux revendications précédentes, dans lequel on obtient en outre au moins une déclaration au sujet d'une qualité de signal de la multiplicité de signaux de mesure, en particulier par une comparaison entre des estimateurs fiables des signaux de mesure des estimateurs paramétriques des signaux de mesure.

**16.** Procédé selon l'une quelconque des trois revendications précédentes, dans lequel on détermine en outre une tendance parmi la multiplicité de signaux de mesure.

**17.** Procédé de fonctionnement d'un appareil de mesure (110) pour la détection d'au moins un analyte dans un liquide corporel selon l'une quelconque des revendications de procédé précédentes, dans lequel on effectue dans ledit au moins un procédé de calibrage un calibrage de signaux de mesure de l'appareil de mesure (110) par rapport à des valeurs de référence de mesures de référence correspondantes, dans lequel on applique une stratégie de calibrage, dans lequel la stratégie de calibrage comprend une ou plusieurs des étapes de procédé suivantes:

- en fonction d'une qualité de calibrage actuelle, on limite une plage utilisable pour une mesure de signaux de mesure de l'appareil de mesure (110), dans lequel, en particulier lors de la détection de signaux de mesure à l'extérieur de la plage couverte par des valeurs de calibrage ou utilisables ou d'une plage de tolérance, qui est basée sur les valeurs de calibrage, on envoie un message à un utilisateur;
- on invite un utilisateur à effectuer de nouveau le procédé de calibrage;
- on informe un utilisateur sur le fait qu'il existe un instant favorable pour l'exécution du procédé de calibrage, en particulier lors d'une dynamique de signal actuellement faible des signaux de mesure et/ou lors d'un important écart à prévoir de la concentration dudit au moins un analyte dans le liquide corporel par rapport à la concentration d'analyte antérieure;
- on invite un utilisateur à effectuer de nouveau le procédé de calibrage à des intervalles réguliers ou irréguliers ou à des instants prédéterminés.

**18.** Programme informatique avec code de programme pour l'exécution du procédé selon l'une quelconque des revendications de procédé précédentes, lorsque le programme est exécuté sur un ordinateur, en particulier sur un ordinateur d'une commande d'un appareil de mesure (110) pour la détection d'au moins un analyte dans un liquide corporel.

**19.** Appareil de mesure (110) pour la détection d'au moins un analyte dans un liquide corporel, en particulier comprenant au moins un détecteur de glucose sanguin à mesure continue (112), dans lequel l'appareil de mesure (110) est conçu pour produire au moins un signal de mesure correspondant à une concentration de l'analyte dans le liquide corporel, dans lequel l'appareil de mesure (110) est en outre conçu pour recevoir des valeurs de référence détectées au moyen d'une mesure de référence indépendante, dans lequel l'appareil de mesure (110) présente au moins une commande (114), en particulier une commande (114) avec au moins un ordinateur, dans lequel la commande (114) est conçue pour exécuter un procédé selon l'une quelconque des revendications de procédé précédentes.

Fig. 1 A

Fig. 1 B

Fig. 1 C

Fig. 1 D

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1154718 B1
- US 6326160 B1
- US 6233471 B
- US 2006264719 A1
- US 20080081977 A1
- US 20080021666 A1
- US 20060281985 A1
- US 7389133 B1
- US 20070016127 A1
- US 20060281985 A

**In der Beschreibung aufgeführte Nicht-Patentliteratur**

- Clinical Chemistry. *Journal of Clinical Chemistry Clinical Biochemistry,* 1983, vol. 21, 709-720
- Exponentielles Glätten. Hartung: Statistik. Lehr- und Handbuch der Angewandten Statistik. 672-673